(19) Europäisches Patentamt
European Patent Office
Office européen des brevets

(11) **EP 0 625 988 B1**

(12) **EUROPEAN PATENT SPECIFICATION**

(45) Date of publication and mention
of the grant of the patent:
**02.01.2003 Bulletin 2003/01**

(51) Int Cl.[7]: **C07K 7/08**, A61K 38/10

(21) Application number: **93923173.4**

(86) International application number:
**PCT/US93/09295**

(22) Date of filing: **28.09.1993**

(87) International publication number:
**WO 94/007914 (14.04.1994 Gazette 1994/09)**

(54) **ALLOSTERIC MODULATORS OF THE NMDA RECEPTOR**

ALLOSTERISCHE MODULATOREN DES NMDA-REZEPTORS

MODULATEURS ALLOSTERIQUES DU RECEPTEUR DE NMDA

(84) Designated Contracting States:
**BE CH DE ES FR GB IE IT LI NL**

(30) Priority: **28.09.1992 US 952818**

(43) Date of publication of application:
**30.11.1994 Bulletin 1994/48**

(73) Proprietor: **MACCECCHINI, Maria-Luisa**
**West Chester, PA 19380 (US)**

(72) Inventor: **MACCECCHINI, Maria-Luisa**
**West Chester, PA 19380 (US)**

(74) Representative: **Brookes Batchellor**
**102-108 Clerkenwell Road**
**London EC1M 5SA (GB)**

(56) References cited:
WO-A-91/01729          WO-A-91/19493
US-A- 5 049 555        US-A- 5 061 721
US-A- 5 086 072

• **Life Sciences, Volume 48, issued 1991, K. WILLIAMS et al.: "Modulation of the NMDA Receptor by Polyamines", pages 469-498, see entire document.**
• **Neuroscience Letters, Volume 118, issued 1990, E.E. MENA et al.: "Conantokin-G: A Novel Peptide Antagonist to the N-Methyl-D-Aspartic Acid (NMDA) Receptor", pages 241-244, see entire document.**
• **The Journal of Biological Chemistry, Volume 265, Number 11, issued 15 April 1990, J.A. HAACK et al.: "Conantokin-T", pages 6025-6029, see entire document.**
• **Science, Volume 249, issued 20 July 1990, B.M. OLIVERA et al.: "Diversity of Conus Neuropeptides", pages 257-263, see entire document.**
• **L.S. GOODMAN et al. (eds.): "The Pharmacological Basis of Therapeutics", published 1975 by Macmillan Publishing Co., Inc. (New York), pages 1-46, see entire document.**

SEQUENCE LISTING

(1) GENERAL INFORMATION:

(i) APPLICANT: Maccecchini, Maria-Luisa

(ii) TITLE OF INVENTION: Allosteric Modulators of the NMDA Receptor and Their Use in the Treatment of CNS Disorders and Enhancement of CNS Function

(iii) NUMBER OF SEQUENCES: 12

(iv) CORRESPONDENCE ADDRESS:

(A) ADDRESSEE: Laurence A. Weinberger
(B) STREET: 882 S. Matlack Street, Ste. 103, P.O. Box 1663
(C) CITY: West Chester
(D) STATE: PA
(E) COUNTRY: USA
(F) ZIP: 19380-0053

(v) COMPUTER READABLE FORM:

    (A) MEDIUM TYPE: Floppy disk
(B) COMPUTER: IBM PC compatible
(C) OPERATING SYSTEM: PC-DOS/MS-DOS
(D) SOFTWARE: Patentin Release #1.0, Version #1.25

(vi) CURRENT APPLICATION DATA:

    (A) APPLICATION NUMBER: US 07/952,818
(B) FILING DATE: 28-SEP-1992
(C) CLASSIFICATION: 514

(viii) ATTORNEY/AGENT INFORMATION:

    (A) NAME: Weinberger, Laurence A.
(B) REGISTRATION NUMBER: 27,965

(ix) TELECOMMUNICATION INFORMATION:

    (A) TELEPHONE: 215-431-1703
(B) TELEFAX: 215-431-4181

(2) INFORMATION FOR SEQ ID NO:1:

(i) SEQUENCE CHARACTERISTICS:

    (A) LENGTH: 17 amino acids
(B) TYPE: amino acid
(D) TOPOLOGY: linear

(ii) MOLECULE TYPE; peptide

(iii) HYPOTHETICAL: NO

(ix) FEATURE:

    (A) NAME/KEY: Modified-site
(B) LOCATION: 3
(D) OTHER INFORMATION: /note= "Amino acid 3 is gamma-carboxyglutamate (Gla)"

(ix) FEATURE:

    (A) NAME/KEY: Modified-site
(B) LOCATION: 4
(D) OTHER INFORMATION: /note= "Amino acid 4 is gamma-carboxyglutamate (Gla)"

(ix) FEATURE:

    (A) NAME/KEY: Modified-site
(B) LOCATION: 7
(D) OTHER INFORMATION: /note= "Amino acid 7 is gamma-carboxyglutamate (Gla)"

(ix) FEATURE:

    (A) NAME/KEY: Modified-site
(B) LOCATION: 10
(D) OTHER INFORMATION: /note= "Amino acid 10 is gamma-carboxyglutamate (Gla)"

(ix) FEATURE:

    (A) NAME/KEY: Modified-site
    (B) LOCATION: 14
    (D) OTHER INFORMATION: /note= "Amino acid 14 is gamma-carboxyglutamate (Gla)"

(ix) FEATURE:

    (A) NAME/KEY: Modified-site
    (B) LOCATION: 17
    (D) OTHER INFORMATION: /note= "Amino acid 17 has an amide rather than a carboxy terminus"

(xi) SEQUENCE DESCRIPTION: SEQ ID NO:1:

```
Gly Glu Glu Glu Leu Gln Glu Asn Gln Glu Leu Ile Arg Glu Lys Ser
1               5                   10                  15

Asn
```

(2) INFORMATION FOR SEQ ID NO:2:

(i) SEQUENCE CHARACTERISTICS:

    (A) LENGTH: 17 amino acids
    (B) TYPE: amino acid
    (D) TOPOLOGY: linear

(ii) MOLECULE TYPE: peptide

(iii) HYPOTHETICAL: NO

(ix) FEATURE:

    (A) NAME/KEY: Modified-site
    (B) LOCATION: 3
    (D) OTHER INFORMATION: /note= "Amino acid 3 is gamma-carboxyglutamate (Gla)"

(ix) FEATURE:

    (A) NAME/KEY: Modified-site
    (B) LOCATION: 4
    (D) OTHER INFORMATION: /note= "Amino acid 4 is gamma-carboxyglutamate (Gla)"

(ix) FEATURE:

    (A) NAME/KEY: Modified-site
    (B) LOCATION: 7
    (D) OTHER INFORMATION: /note= "Amino acid 7 is gamma-carboxyglutamate (Gla)"

(ix) FEATURE:

    (A) NAME/KEY: Modified-site
    (B) LOCATION: 10
    (D) OTHER INFORMATION: /note= "Amino acid 10 is gamma-carboxyglutamate (Gla)"

(ix) FEATURE:

(A) NAME/KEY: Modified-site
(B) LOCATION: 14
(D) OTHER INFORMATION: /note= "Amino acid 14 is gamma-carboxyglutamate (Gla)"

(xi) SEQUENCE DESCRIPTION: SEQ ID NO:2:

```
Gly Glu Glu Glu Leu Gln Glu Asn Gln Glu Leu Ile Arg Glu Lys Ser
1               5                   10                  15

Asn
```

(2) INFORMATION FOR SEQ ID NO:3:

(i) SEQUENCE CHARACTERISTICS:

(A) LENGTH: 18 amino acids
(B) TYPE: amino acid
(D) TOPOLOGY: linear

(ii) MOLECULE TYPE: peptide

(iii) HYPOTHETICAL: NO

(ix) FEATURE:

(A) NAME/KEY: Modified-site
(B) LOCATION: 4
(D) OTHER INFORMATION: /note= "Amino acid 4 is gamma-carboxyglutamate (Gla)"

(ix) FEATURE:

(A) NAME/KEY: Modified-site
(B) LOCATION: 5
(D) OTHER INFORMATION: /note= "Amino acid 5 is gamma-carboxyglutamate (Gla)"

(ix) FEATURE:

(A) NAME/KEY: Modified-site
(B) LOCATION: 8
(D) OTHER INFORMATION: /note= "Amino acid 8 is gamma-carboxyglutamate (Gla)"

(ix) FEATURE:

(A) NAME/KEY: Modified-site
(B) LOCATION: 11
(D) OTHER INFORMATION: /note= "Amino acid 11 is gamma-carboxyglutamate (Gla)"

(ix) FEATURE:

(A) NAME/KEY: Modified-site
(B) LOCATION: 15
(D) OTHER INFORMATION: /note= "Amino acid 15 is gamma-carboxyglutamate (Gla)"

(ix) FEATURE:

(A) NAME/KEY: Modified-site
(B) LOCATION: 18
(D) OTHER INFORMATION: /note= "Amino acid 18 has an amide rather than a carboxy terminus"

(xi) SEQUENCE DESCRIPTION: SEQ ID NO:3:

```
Tyr Gly Glu Gla Gla Leu Gln Glu Asn Gln Glu Leu Ile Arg Glu Lys
1               5                   10                  15

Ser Asn
```

(2) INFORMATION FOR SEQ ID NO:4:

(i) SEQUENCE CHARACTERISTICS:

(A) LENGTH: 17 amino acids
(B) TYPE: amino acid
(D) TOPOLOGY: linear

(ii) MOLECULE TYPE: peptide

(iii) HYPOTHETICAL: NO

(ix) FEATURE:

(A) NAME/KEY: Modified-site
(B) LOCATION: 1
(D) OTHER INFORMATION: /note= "Amino acid 1 is acetylated"

(ix) FEATURE:

(A) NAME/KEY: Modified-site
(B) LOCATION: 3
(D) OTHER INFORMATION: /note= "Amino acid 3 is gamma-carboxyglutamate (Gla)"

(ix) FEATURE:

(A) NAME/KEY: Modified-site
(B) LOCATION: 4
(D) OTHER INFORMATION: /note= "Amino acid 4 is gamma-carboxyglutamate (Gla)"

(ix) FEATURE:

(A) NAME/KEY: Modified-site
(B) LOCATION: 7
(D) OTHER INFORMATION: /note= "Amino acid 7 is gamma-carboxyglutamate (Gla)"

(ix) FEATURE:

(A) NAME/KEY: Modified-site
(B) LOCATION: 10
(D) OTHER INFORMATION: /note= "Amino acid 10 is gamma-carboxyglutamate (Gla)"

(ix) FEATURE:

(A) NAME/KEY: Modified-site
(B) LOCATION: 14
(D) OTHER INFORMATION: /note= "Amino acid 14 is gamma-carboxyglutamate (Gla)"

(ix) FEATURE:

(A) NAME/KEY: Modified-site
(B) LOCATION: 17
(D) OTHER INFORMATION: /note= "Amino acid 17 has an amide rather than a carboxy terminus"

(xi) SEQUENCE DESCRIPTION: SEQ ID NO:4:

```
Gly Glu Glu Glu Leu Gln Glu Asn Gln Glu Leu Ile Arg Glu Lys Ser
1               5                   10                  15

Asn
```

(2) INFORMATION FOR SEQ ID NO:5:

(i) SEQUENCE CHARACTERISTICS:

(A) LENGTH: 17 amino acids
(B) TYPE: amino acid
(D) TOPOLOGY: linear

(ii) MOLECULE TYPE: peptide

(iii) HYPOTHETICAL: NO

(ix) FEATURE:

(A) NAME/KEY: Modified-site
(B) LOCATION: 17
(D) OTHER INFORMATION: /note= "Amino acid 17 has an amide rather than a carboxy terminus"

(xi) SEQUENCE DESCRIPTION: SEQ ID NO:5:

```
Gly Glu Glu Glu Leu Gln Glu Asn Gln Glu Leu Ile Arg Glu Lys Ser
1               5                   10                  15

Asn
```

(2) INFORMATION FOR SEQ ID NO:6:

(i) SEQUENCE CHARACTERISTICS:

(A) LENGTH: 18 amino acids
(B) TYPE: amino acid
(D) TOPOLOGY: linear

(ii) MOLECULE TYPE: peptide

(iii) HYPOTHETICAL: NO

(ix) FEATURE:

(A) NAME/KEY: Modified-site
(B) LOCATION: 18
(D) OTHER INFORMATION: /note= "Amino acid 18 has an amide rather than a carboxy terminus"

(xi) SEQUENCE DESCRIPTION: SEQ ID NO:6:

```
Tyr Gly Glu Glu Glu Leu Gln Glu Asn Gln Glu Leu Ile Arg Glu Lys
1               5                   10                  15

Ser Asn
```

(2) INFORMATION FOR SEQ ID NO:7:

    (i) SEQUENCE CHARACTERISTICS:

        (A) LENGTH: 6 amino acids
        (B) TYPE: amino acid
        (D) TOPOLOGY: linear

    (ii) MOLECULE TYPE: peptide

    (iii) HYPOTHETICAL: NO

    (ix) FEATURE:

        (A) NAME/KEY: Modified-site
        (B) LOCATION: 6
        (D) OTHER INFORMATION: /note= "Amino acid 6 has an amide rather than a carboxy terminus"

    (xi) SEQUENCE DESCRIPTION: SEQ ID NO:7:

```
Ile Arg Glu Lys Ser Asn
1               5
```

(2) INFORMATION FOR SEQ ID NO:8:

    (i) SEQUENCE CHARACTERISTICS:

        (A) LENGTH: 9 amino acids
        (B) TYPE: amino acid
        (D) TOPOLOGY: linear

    (ii) MOLECULE TYPE: peptide

    (iii) HYPOTHETICAL: NO

    (ix) FEATURE:

        (A) NAME/KEY: Modified-site
        (B) LOCATION: 9
        (D) OTHER INFORMATION: /note= "Amino acid 9 has an amide rather than a carboxy terminus"

    (xi) SEQUENCE DESCRIPTION: SEQ ID NO:8:

```
Gln Glu Leu Ile Arg Glu Lys Ser Asn
1               5
```

(2) INFORMATION FOR SEQ ID NO:9:

    (i) SEQUENCE CHARACTERISTICS:

        (A) LENGTH: 12 amino acids
        (B) TYPE: amino acid
        (D) TOPOLOGY: linear

    (ii) MOLECULE TYPE: peptide

    (iii) HYPOTHETICAL: NO

    (ix) FEATURE:

        (A) NAME/KEY: Modified-site
        (B) LOCATION: 12
        (D) OTHER INFORMATION: /note= "Amino acid 12 has an amide rather than a carboxy terminus"

    (xi) SEQUENCE DESCRIPTION: SEQ ID NO:9:


                Gln Glu Asn Gln Glu Leu Ile Arg Glu Lys Ser Asn
                1               5                   10


(2) INFORMATION FOR SEQ ID NO:10:

    (i) SEQUENCE CHARACTERISTICS:

        (A) LENGTH: 16 amino acids
        (B) TYPE: amino acid
        (D) TOPOLOGY: linear

    (ii) MOLECULE TYPE: peptide

    (iii) HYPOTHETICAL: NO

    (ix) FEATURE:

        (A) NAME/KEY: Modified-site
        (B) LOCATION: 16
        (D) OTHER INFORMATION: /note= "Amino acid 16 has an amide rather than a carboxy terminus"

    (xi) SEQUENCE DESCRIPTION: SEQ ID NO:10:


            Glu Glu Glu Leu Gln Glu Asn Gln Glu Leu Ile Arg Glu Lys Ser Asn
            1               5                   10                  15


(2) INFORMATION FOR SEQ ID NO:11:

    (i) SEQUENCE CHARACTERISTICS:

        (A) LENGTH: 17 amino acids
        (B) TYPE: amino acid
        (D) TOPOLOGY: linear

    (ii) MOLECULE TYPE: peptide

    (iii) HYPOTHETICAL: NO

    (ix) FEATURE:

(A) NAME/KEY: Modified-site
(B) LOCATION: 3
(D) OTHER INFORMATION: /note= "Amino acid 3 is D-glutamate (D-Glu)"

(ix) FEATURE:

(A) NAME/KEY: Modified-site
(B) LOCATION: 4
(D) OTHER INFORMATION: /note= "Amino acid 4 is D-glutamate (D-Glu)"

(ix) FEATURE:

(A) NAME/KEY: Modified-site
(B) LOCATION: 7
(D) OTHER INFORMATION: /note= "Amino acid 7 is D-glutamate (D-Glu)"

(ix) FEATURE:

(A) NAME/KEY: Modified-site
(B) LOCATION: 10
(D) OTHER INFORMATION: /note= "Amino acid 10 is D-glutamate (D-Glu)"

(ix) FEATURE:

(A) NAME/KEY: Modified-site
(B) LOCATION: 14
(D) OTHER INFORMATION: /note= "Amino acid 14 is D-glutamate (D-Glu)"

(ix) FEATURE:

(A) NAME/KEY: Modified-site
(B) LOCATION: 17
(D) OTHER INFORMATION: /note= "Amino acid 17 has an amide rather than a carboxy terminus"

(xi) SEQUENCE DESCRIPTION: SEQ ID NO:11:

```
Gly Glu Glu Glu Leu Gln Glu Asn Gln Glu Leu Ile Arg Glu Lys Ser
1               5                   10                  15

Asn
```

(2) INFORMATION FOR SEQ ID NO:12:

(i) SEQUENCE CHARACTERISTICS:

(A) LENGTH: 21 amino acids
(B) TYPE: amino acid
(D) TOPOLOGY: linear

(ii) MOLECULE TYPE: peptide

(iii) HYPOTHETICAL: NO

(ix) FEATURE:

(A) NAME/KEY: Modified-site
(B) LOCATION: 3

(D) OTHER INFORMATION: /note= "Amino acid 3 is gamma-carboxyglutamate (Gla)"

(ix) FEATURE:

    (A) NAME/KEY: Modified-site
    (B) LOCATION: 4
    (D) OTHER INFORMATION: /note= "Amino acid 4 is gamma-carboxyglutamate"

(ix) FEATURE:

    (A) NAME/KEY: Modified-site
    (B) LOCATION: 10
    (D) OTHER INFORMATION: /note= "Amino acid 10 is gamma-carboxyglutamate"

(ix) FEATURE:

    (A) NAME/KEY: Modified-site
    (B) LOCATION: 14
    (D) OTHER INFORMATION: /note= "Amino acid 14 is gamma-carboxyglutamate"

(ix) FEATURE:

    (A) NAME/KEY: Modified-site
    (B) LOCATION: 21
    (D) OTHER INFORMATION: /note= "Amino acid 21 has an amide rather than a carboxy terminus"

(xi) SEQUENCE DESCRIPTION: SEQ ID NO:12:

```
Gly Glu Glu Glu Tyr Gln Lys Met Leu Glu Asn Leu Arg Glu Ala Glu
1               5                   10                  15

Val Lys Lys Asn Ala
            20
```

## Description

### Technical Field

This invention relates to the NMDA subtype of the glutamate receptor and more specifically to agonists, partial agonists, and antagonists of a polyamine associated recognition site which may be used to modulate the response of the receptor in order to ameliorate certain neurological, neuropsychological and neuropsychiatric conditions, prevent neurodegeneration after CNS trauma and injury, as well as to enhance/affect learning and memory.

### Disclosure of Invention

During the past twenty years a revolution in understanding the basic structure and chemistry of the synaptic interconnections of neural tissues has taken place which promises hope that such understanding will permit treatment of neural tissue damage and disorders. These studies have centered around an understanding of the properties of the neurochemical transmitters released from presynaptic membranes and, most importantly, the postsynaptic receptors for the transmitters. The nicotinic acetylcholine and GABA receptors have been characterized and found to be under the control of allosteric modulators. During the past ten years a great deal of attention has been directed to the excitatory amino acids (EAAs), principally glutamate and aspartate, and their receptors since they are thought to mediate fast excitatory transmission in the mammalian central nervous system. EAA receptors are broken down into two major subtypes: ionotropic and metabotropic. The ionotropic receptors contain ligand-gated ion channels and mediate ion fluxes for signalling, while the metabotropic receptors use G proteins for signalling. Classification of the ionotropic EAA receptors is based upon the agonists (stimulators) that selectively activate them. Presently, it is believed that there are three major subtypes: a receptor responsive to N-methyl-D-aspartate (NMDA receptors) and two receptors not responsive to NMDA but responsive to $\alpha$-amino-3-hydroxy-5-methyl-4-isoxazoleproprionic acid (AMPA) and to kainate.

The NMDA receptor controls the flow of both divalent ($Ca^{++}$) and monovalent ($Na^+$, $K^+$) ions into the postsynaptic neural cell although it is the $CA^{++}$ flux which is of the greatest interest (reviewed in Foster and Fagg, 1987; Mayer and Miller, 1990). The NMDA receptor has been implicated during development in specifying neuronal architecture and synaptic connectivity, and may be involved in experience dependent synaptic modifications. In addition, NMDA receptors are also thought to be involved in long term potentiation, CNS plasticity, cognitive processes, memory acquisition, and learning. Furthermore, the NMDA receptor has also drawn particular interest since it appears to be involved in a broad spectrum of CNS disorders. For instance, during brain ischemia caused by stroke or traumatic injury, excessive amounts of the excitatory amino acid glutamate are released from damaged or oxygen deprived neurons. This excess glutamate binds to the NMDA receptors which opens their ligand-gated ion channels thereby allowing calcium influx producing a high level of intracellular calcium which activates a biochemical cascade resulting in protein degradation and cell death. This phenomenon, known as excitotoxicity, is also thought to be responsible for the neurological damage associated with other disorders ranging from hypoglycemia and cardiac arrest to epilepsy. In addition, there are preliminary reports indicating similar involvement in the chronic neurodegeneration of Huntington's, Parkinson's, and Alzheimer's diseases. Activation of the NMDA receptor has been shown to be responsible for post-stroke convulsions, and, in certain models of epilepsy, activation of the NMDA receptor has been shown to be necessary for the generation of seizures.

Neuropsychiatric involvement of the NMDA receptor has also been recognized since blockage of the NMDA receptor $Ca^{++}$ channel by the animal anesthetic PCP (phencyclidine) produces a psychotic state in humans similar to schizophrenia (reviewed in Johnson, K. and Jones, S., 1990). Further, NMDA receptors have also been implicated in certain types of spatial learning. Interestingly, both the spatial and temporal distribution of NMDA receptors in mammalian nervous systems have been found to vary. Thus, cells may produce NMDA receptors at different times in their life cycles and not all CNS neural cells may utilized the NMDA receptor.

Drugs acting on the NMDA receptor are, therefore, expected to have enormous therapeutic potential. For instance, Cordi et al. in U.S. Patent No. 4,904,681 teach the use of a compound, D-Cycloserine, which modulates the NMDA receptor to improve/enhance memory and to treat cognitive deficits linked to a neurological disorder. In U.S. Patent No. 5,061,721 Cordi et al. teach the use of a combination of D-cycloserine and D-alanine to treat Alzheimer's disease, age-associated memory impairment, learning deficits, and psychotic disorders as well as to improve memory or learning in healthy individuals. Trullas et al. in U.S. Patent 5,086,072 teach the use of another compound, 1-aminocyclopropanecarboxylicacid (ACPC) which modulates the NMDA receptor, to treat mood disorders including major depression, bipolar disorder, dysthemia and seasonal effective disorder. Trullas also teaches that ACPC mimics the actions of clinically effective antidepressants in animal models. In addition, Trullas teaches by reference to a copending U.S. patent application, that ACPC and its derivatives may be used to treat neuropharmacological disorders resulting from excessive activation of the NMDA receptor.

The NMDA receptor is believed to consist of several protein chains embedded in the postsynaptic membrane.

The two first types of subunits discovered so far form a large extracellular region which probably contains most of the allosteric binding sites, several transmembrane regions looped and folded so as to form a pore or channel which is permeable to $Ca^{++}$, and a carboxyl terminal region with an as yet unknown function. The opening and closing of the channel is regulated by the binding of various ligands (allosteric modulators) to domains of the protein residing on the extracellular surface. The binding of the allosteric modulators is thought to affect a conformational change in the overall structure of the protein which is ultimately reflected in the channel opening, partially opening, partially closing, or closing.

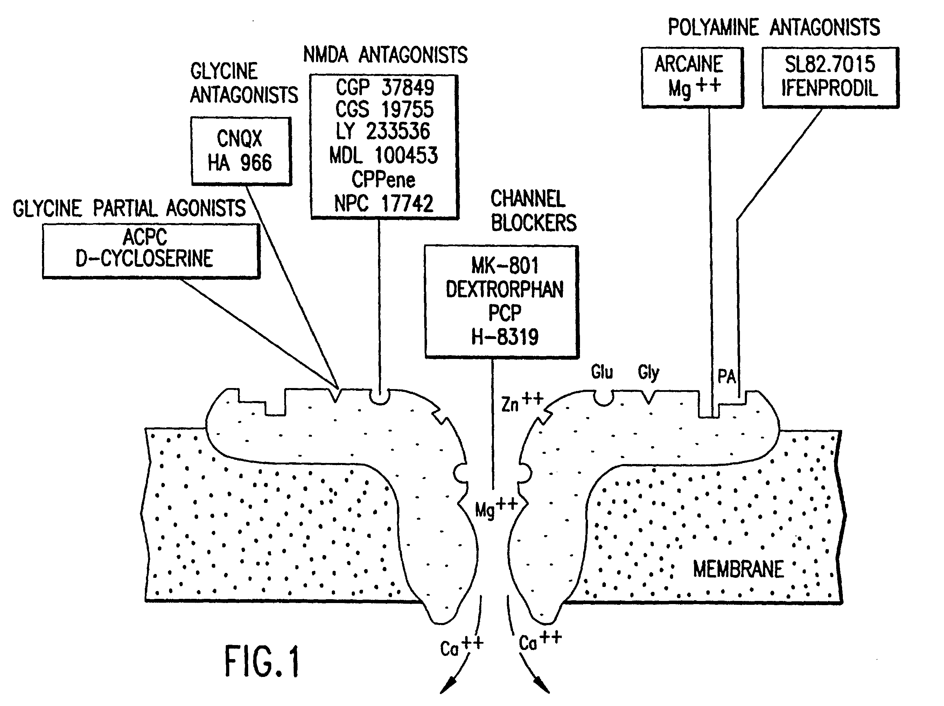

Figure 1 is a representation of the NMDA receptor showing schematically the principal recognition/binding sites which had been elucidated prior to the present invention. The site marked *Glu* is the receptor site for the principal excitatory amino acid neurotransmitter, glutamate. This site also binds NMDA. Binding of glutamate/NMDA has been shown to stimulate the flow of $CA^{++}$ through the channel. Thus, glutamate is said to have an agonist (stimulatory) activity. Several competitive inhibitors of the actions of glutamate also bind at this site and include those identified in the box in Figure 1 labeled *NMDA Antagonists.* Since these competitive inhibitors of the glutamate site block the flow of $CA^{++}$ through the channel, they are said to have an antagonist activity .

Binding to the glutamate site of the NMDA receptor alone is necessary, but not sufficient, to open the cation channel. It has been found that glycine must also be bound to the receptor for the channel to open. Thus, glycine is also an NMDA receptor agonist. Since glycine binding is not inhibited by glutamate or by competitive inhibitors of glutamate, it is believed that glycine does not bind at the same site as glutamate. Thus ,a second and separate binding site for glycine has been proposed as is indicated in Figure 1 by *Gly*. The ligand-gated ion channel of the NMDA receptor is, thus, under the control of at least two distinct allosteric modulatory sites. Competitive inhibitors of the glycine site are also known and are identified in the box in Figure 1 labeled *Glycine Antagonists*. The inhibitors are also NMDA receptor antagonists.

Recently a class of compounds (ACPC and derivatives thereof) has been discovered which bind to the glycine site with a high affinity and which possess an agonist activity in the absence of glycine. However, the degree of agonist activity (NMDA receptor channel opening) achieved with this class of compounds is less than that observed when glycine binds to the same site. Conversely, when these compounds are presented to the receptor in the presence of glycine, they reduce the degree of channel opening since they bind to the glycine site in place of glycine and are less effective agonists than glycine. Thus, these compounds are partial agonists at the glycine site that act as functional antagonists and may be used to reduce the net $Ca^{++}$ flux through the NMDA receptor channel. Cordi et al. also teach that D-cycloserine interacts with the glycine site and is an agonist in the absence of glycine and an antagonist in the presence of glycine thereby again displaying for D-cycloserine the pattern characteristic of a partial agonist.

Several compounds are known which are antagonistic to the flow of cations through the NMDA receptor but which do not competitively inhibit the binding of allosteric modulators to any of the known modulatory sites. Instead, these compounds bind inside the open cation channel and are generally known as channel blockers. These are shown in Figure 1 in the box labeled *Channel Blockers*. In fact, binding of the channel blocker [3H]MK-801 is a good measure of the activation of the NMDA receptor complex. When the channel is open, [3H]MK-801 may freely pass into the channel and bind to its recognition site in the channel. Conversely, when the channel is closed, [3H]MK-801 may not freely pass into the channel and bind. When the channel is partially open (partially closed) less [3H]MK-801 is able to bind than when the channel is fully open.

It should be understood that it is believed that the channel is in constant motion, alternating between a cation passing (open) and a cation blocking (closed) state. it is not known at present whether the allosteric modulators actually increase the time during which the channel is open to the flow of ions, or whether the modulators increase the frequency of opening. Both effects might be occurring at the same time. Thus, the terms open and close or agonistic or antagonistic refer to a time averaged affect.

$Mg^{++}$ and $Zn^{++}$ also modulate the NMDA receptor. The exact location of the divalent cation binding sites is still unclear. $Zn^{++}$ appears to be antagonist to channel opening and appears to bind to an extracellular domain. $Mg^{++}$ shows a biphasic activation curve at low concentrations, is an agonist for NMDA receptor function except at high concentrations at which it is an antagonist, and appears to be absolutely necessary for proper receptor functioning. It appears to bind at two sites. There appears to be a voltage dependant binding site for $Mg^{++}$ within the channel and another binding site on the extracellular domain. These sites are indicated in Figure 1 by *$Mg^{++}$* and *$Zn^{++}$*.

Recently, (Ransom and Stec, 1988; Reynolds and Miller, 1989; reviewed in Williams et al., 1992) a third class of agonists which modulate the excitatory synaptic transmission at the NMDA receptor has been identified. These agonists are polyamines, principally the endogenous polyamines spermine and spermidine, which bind to another extracellular allosteric modulatory site on the NMDA receptor. In Figure 1 the polyamine binding site is labeled *PA*. The polyamines do not bind to the glutamate/NMDA site, the glycine site, or the channel blocker sites. There may be some relation between the extracellular $Mg^{++}$ binding site and the polyamine site but this relationship has not yet been fully elucidated. There is strong evidence accumulating that, like glycine, polyamine binding is necessary, but not sufficient, for maximum functioning/activation of the NMDA receptor coupled cation channel. In fact, the polyamine site may be the most complex allosteric modulatory site yet identified. There appears to be a broad range of polyamine (diamine, triamine, and tetraamine) compounds which will modulate the site, some of which appear to be agonists, others partial agonists, and, finally, some antagonists. The exact structural/chemical requirements for the site are, thus, not completely understood. In addition, the site appears to be capable of a negative modulation; that is, binding of a particular compound (DA10) decreases rather than increases the channel opening. This activity has been termed inverse agonist activity. Such inverse agonist binds competitively at the same site as an agonist but produces the opposite effect as the agonist.

Much of what has been learned about the allosteric modulation of the NMDA receptor has been made possible by the discovery of compounds which blocked one or another action of the various modulatory agents. The approach of using blocking agents to map pathways has a long history in biochemical and biophysical research and often these blocking agents have been discovered to be useful therapeutic agents. During the past few years a number of unusual toxins composed of a variety of polypeptides have been identified in the paralytic venoms of the fish hunting cone snails of the genus *Conus* found in the Philippine archipelago. Many of these peptides have been discovered to affect ion channel function. Conotoxins include the paralytic a, $\mu$, and w conotoxins which block nicotinic acetylcholine receptors, sodium channels, and voltage sensitive calcium channels respectively (reviewed in Olivera, et al., 1990). Toxins from two of the snails *Conus tulipa* and *Conus geographus* have particularly unique compositions since they contain the unusual amino acid $\gamma$-carboxyglutamate, Conantokin-T (SEQ ID NO: 12) (21 amino acids from *tulipas*) containing 4 and Conantokin-G (SEQ ID NO: 1) (17 amino acids from *geographus*) containing 5. The sequence of these amino acids is shown below with the $\gamma$-carboxyglutamate shown in bold:

Conantokin-G:

Gly-Glu-**Gla-Gla**-Leu-Gln-**Gla**-Asn-Gln-**Gla**-Leu-Ile-Arg-**Gla**-Lys-Ser-Asn-NH$_2$  (SEQ ID NO: 1)

Conantokin-T:

Gly-Glu-**Gla-Gla**-Tyr-Gln-Lys-Met-Leu-**Gla**-Asn-Leu-Arg-**Gla**-Ala-Glu-Val-Lys-Lys-Ans-Ala-NH$_2$  (SEQ ID NO: 12)

Recently both conantokin-T (SEQ ID NO: 12) and conantokin-G (SEQ ID NO: 1) were reported to act as NMDA receptor antagonists. (Haack et al., 1990; Mena et al., 1990) The toxins were shown to decrease NMDA induced increases of intracellular $Ca^{++}$ levels and to block NMDA induced increases in cGMP levels in both primary neuronal cell cultures and brain slice preparations. Mena et al. (1990) noted that the mechanism of antagonism of Conantokin-G (SEQ ID NO: 1) appeared to be different from previously described competitive or non-competitive NMDA receptor antagonists. However, none of this initial work elucidated the mechanism(s) underlying the antagonism of Conantokin-G (SEQ ID NO: 1) or Conantokin-T (SEQ ID NO: 12) to NMDA receptor response.

## Summary of the Invention

It has been discovered that Con-G (SEQ ID NO: 1) acts as an antagonist of the NMDA receptor through its action as a potent, selective, and non-competitive inhibitor of the polyamine modulated responses with a neurochemical profile which is distinct from previously described polyamine antagonists. Con-G (SEQ ID NO: 1) binds at a unique and heretofore unidentified site on the extracellular domains of the NMDA receptor. Further, a new class of allosteric modulators has been discovered, which are derived from Con-G (SEQ ID NO: 1) and act as partial or full agonists of the NMDA receptor at the polyamine site or a site associated with the polyamine site. One of these compounds, a six peptide unit, is almost as effective an agonist for the polyamine site (or polyamine associated site) as any known polyamine is for the polyamine site. Further, this derivative demonstrates that it is the carboxyl end of the Con-G derivatives which is responsible for their agonist properties at the polyamine or polyamine associated site.

Thus, new classes of allosteric modulators both of the polyamine site and of a plyamine associated site of the glutamate subtype of the NMDA receptor have been discovered which exhibit a range of activities from complete antagonism, to partial agonism, to full agonism. This class of compounds may be used to modulate the flow of $Ca^{++}$ through the NMDA receptor. The range of modulation of the compounds acting at the polyamine associated site goes from partially inhibitory (down modulatory) to partially stimulatory (up modulatory). Depending on the required activity, compounds from this class may be used as pharmaceutical neuroprotectants to treat acute cases of massive Ca$^{++}$ influx due to CNS injury and trauma, as well as to treat convulsions, mood disorders, and other neuropsychiatric and neurodegenerative diseases due to chronic disturbances in control of Ca$^{++}$ influx. Similarly, compounds of this class can be selected for the required activity to treat cognitive disorders and to enhance memory and learning.

According to the present invention there is provided a peptide selected from the group consisting of a peptide selected from the group consisting of:

(i) Tyr-Gly-Glu-Glu-Glu-Leu-Gln-Gla-Asn-Gln-Gla-Leu-Ile-Arg-Gla-Lys-Ser-Asn-NH$_2$ (SEQ ID NO:3);

(ii) Gly-Glu-Glu-Glu-Leu-Gln-Glu-Asn-Gln-Glu-Leu-Ile-Arg-Glu-Lys-Ser-Asn-NH$_2$ (SEQ ID NO:5);

(iii) Tyr-Gly-Glu-Glu-Glu-Leu-Gln-Glu-Asn-Gln-Glu-Leu-Ile-Arg-Glu-Lys-Ser-Asn-NH$_2$ (SEQ ID NO:6);

(iv) Ile-Arg-Glu-Lys-Ser-Asn-NH$_2$ (SEQ ID NO:7);

(v) Gln-Glu-Leu-Ile-Arg-Glu-Lys-Ser-Asn-NH$_2$ (SEQ ID NO:8);

(vi) Gln-Glu-Asn-Gln-Glu-Leu-Ile-Arg-Glu-Lys-Ser-Asn-NH$_2$ (SEQ ID NO:9);

(vii) Glu-Glu-Glu-Leu-Gln-Glu-Asn-Gln-Glu-Leu-Ile-Arg-Glu-Lys-Ser-Asn-NH$_2$ (SEQ ID NO:10);

(viii) Gly-Glu-DGlu-DGlu-Leu-Gln-Glu-Asn-Gln-DGlu-Leu-Ile-Arg-DGlu-Lys-Ser-Asn-NH$_2$ (SEQ ID NO:11).

Suitably, a pharmaceutical composition is provided comprising the peptide and a pharmaceutically acceptable carrier.

Preferably, the peptide is used in the manufacture of a medicament for the treatment of a neuropsychopharmacological disorder, a neurodegenerative disorder, a chemical toxicity or a cognitive deficit.

The present invention also provides the use of a peptide as set forth above for the manufacture of a medicament for the enhancement of memory and associated mental processes.

There is also provided the use of Con-G (SEQ ID NO:1) in the manufacture of a medicament for treating excitotoxicity resulting from over-stimulation of the NM13A receptor, in particular wherein said excitotoxicity is associated with epilepsy or epileptic seizure.

Figure 1 shows a schematic representation of the known binding sites on the NMDA receptor indicating both the sites at which stimulatory agonists and inhibirotry antagonists bind.

Figure 2 is a plot showing the stimulation of the NMDA receptor in well washed forebrain membranes by polyamines as indicated by increased [$^3$H]MK-801 binding to the receptor in the nominal absence of endogenous glutamate and glycine. Figure 2(A) shows the response to spermine, while Figure 2(B) shows the response to spermidine.

Figure 3 shows the same plots as in Figure 2 with the addition of curves representing the [³H]MK-801 binding when 400 nM, 800 nM, and 1200 nM Con-G (SEQ ID NO: 1) is added.

Figure 4 shows a plot of the concentration dependent inhibition of the maximum stimulation of [³H]MK-801 binding to brain membranes produced by spermine and spermidine (at 50μM concentration each) as the concentration of Con-G (SEQ ID NO: 1) increases.

Figure 5 shows the effect of increasing concentrations of Con-G (SEQ ID NO: 1) upon basal [³H]MK-801 binding and glycine (10 μM) and glutamate (10 μM) stimulated [³H]MK-801 binding to brain membranes. The curves preceding the break (⊣⊢) represent [³H]MK-801 binding in the absence of Con-G (SEQ ID NO: 1).

Figure 6 shows the effect of 10 μM Con-G (SEQ ID NO: 1) on glycine stimulated [³H]MK-801 binding to brain membranes as a function of increasing glycine concentration. Con-G (SEQ ID NO: 1) modestly inhibits glycine enhanced [³H]MK-801 binding by about 15% with no remarkable affect on its potency. The dotted line represents a theoretical binding curve if the combination of Con-G (SEQ ID NO: 1) and glycine was additive.

Figure 7 shows the schematic representation of the allosteric modulatory sites on the NMDA receptor of Figure 1 to which has been added a schematic representation of the newly discovered allosteric modulatory site at which Con-G (SEQ ID NO: 1) affects its action.

Figure 8 shows the effect of increasing concentrations of Con-G (SEQ ID NO: 1), Glu Con-G (SEQ ID NO: 5), Con-G OH (SEQ ID NO: 2), NAc Con-G (SEQ ID NO: 4), Tyr⁰ Con-G (SEQ ID NO: 3), and Glu Con-G (12-17) (SEQ ID NO: 7) on the maximum spermine (25 μM) induced enhancement of [³H]MK-801 binding to the NMDA receptor in well washed brain membranes.

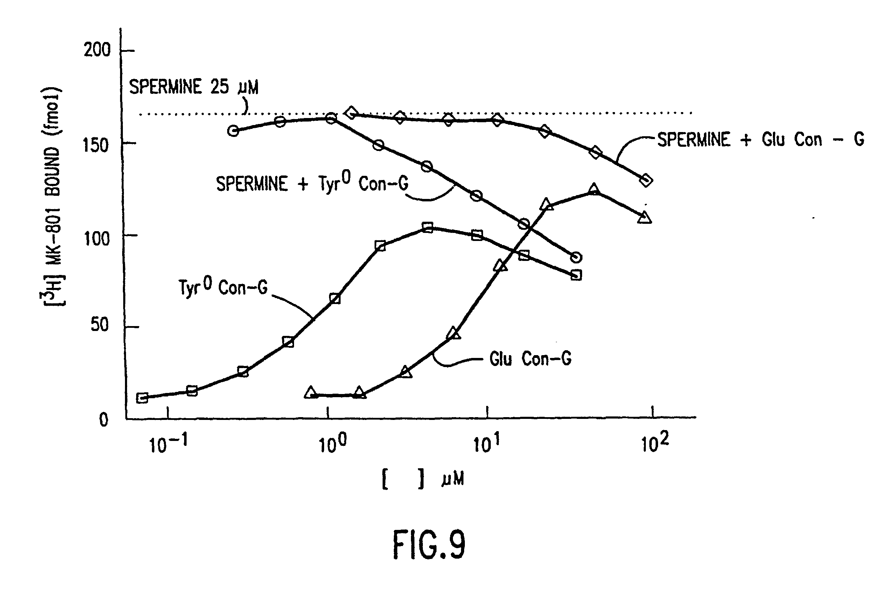

Figure 9 shows the effect of Glu Con-G (SEQ ID NO:5) and Tyr⁰ Con-G (SEQ ID NO: 3) on [³H]MK-801 binding to a well washed brain membrane preparation in the presence and absence of the polyamine spermine (25 μM). The dotted line represents the level of binding achieved with a maximally effective concentration of spermine, 25 μM. The top two curves show that at higher concentrations Glu Con-G (SEQ ID NO:5) and Tyr⁰ Con-G (SEQ ID NO: 3) are antagonistic to spermine induced enhancement of [³H]MK-801 binding. The bottom two curves show that in the absence of spermine, both Glu Con-G (SEQ ID NO: 5) and Tyr⁰ Con-G (SEQ ID NO: 3) are agonists which enhance [³H]MK-801 binding. This dual behavior is typical of partial agonists.

Figure 10 shows the concentration dependent enhancement by Tyr⁰ Con-G (SEQ ID NO: 3), Glu Con-G (SEQ ID NO: 5), Glu Con-G (12-17) (SEQ ID NO: 7), Glu Con-G (2-17) (SEQ ID NO: 10), Tyr⁰ Glu Con-G (SEQ ID NO: 6), Glu Con-G (6-17) (SEQ ID NO: 9), and Glu Con-G (9-17) (SEQ ID NO: 8) of [³H]MK-801 binding to well washed brain membranes in the absence of polyamines.

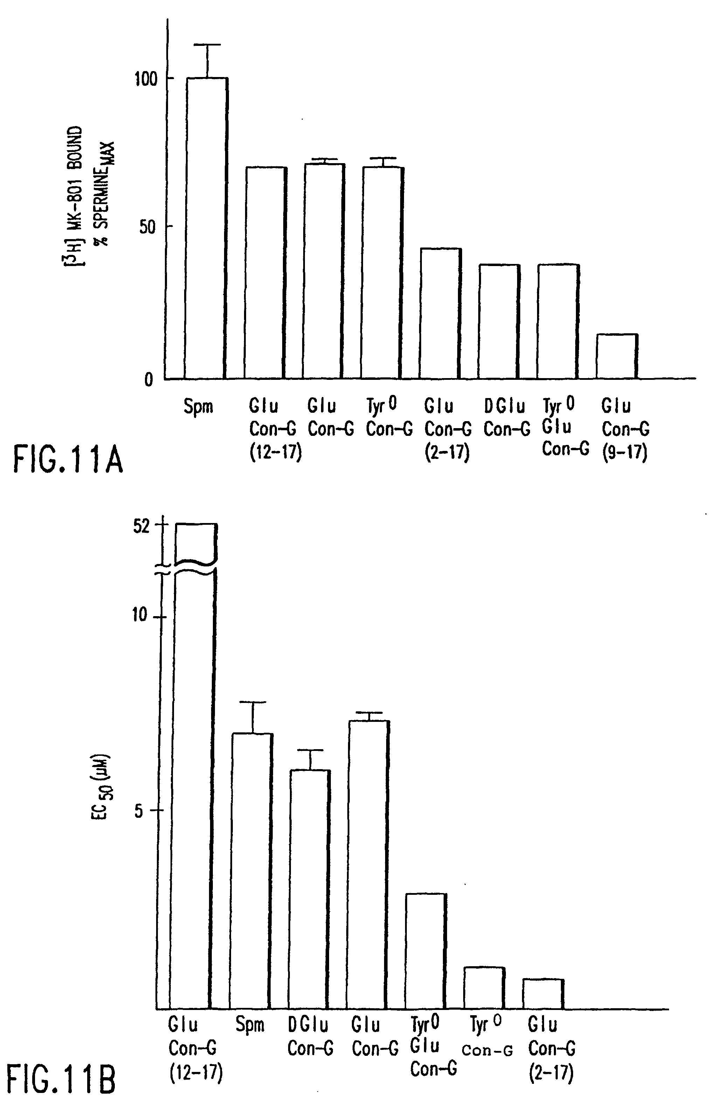

Figure 11(A) shows the maximum enhancement (efficacy) of [³H]MK-801 binding to the NMDA receptor in well washed brain membranes achieved with Glu Con-G (12-17) (SEQ ID NO: 7), Glu Con-G (SEQ ID NO: 5), Tyr⁰ Con-G (SEQ ID NO: 3), Glu Con-G (2-17) (SEQ ID NO: 10), D-Glu Con-G (SEQ ID NO: 11), Tyr⁰ Glu Con-G (SEQ ID NO: 6), and Glu Con-G (9-17) (SEQ ID NO: 8) as a percentage of the maximum enhancement produced by the polyamine spermine. The potencies ($EC_{50}$) of Glu Con-G (12-17) (SEQ ID NO: 7), Spermine, D-Glu Con-G (SEQ ID NO: 11), Glu Con-G (SEQ ID NO: 5), Tyr⁰ Glu Con-G (SEQ ID NO: 6), Tyr⁰ Con-G (SEQ ID NO: 3), and Glu Con-G (2-17) (SEQ ID NO: 10) are shown in Figure 11(B).

Figure 12 shows the noncompetitive inhibition of Glu Con-G (SEQ ID NO:5) enhanced [³H]MK-801 binding to well washed brain membranes by increasing concentrations of Con-G (SEQ ID NO: 1).

Figure 13 shows the inhibition of spermine (18.3 μM), Glu Con-G (SEQ ID NO: 5) (10 μM), and Mg⁺⁺ (100 μM) enhanced [³H]MK-801 binding to well washed brain membranes as a function of increasing concentrations of Con-G (SEQ ID NO: 1). The dotted line shows basal (unstimulated) binding.

Figure 14(A) shows the effect of increasing concentrations of the polyamine antagonist, arcaine, on spermine enhanced [³H]MK-801 binding to well washed brain membranes. Figure 14(B) shows the effect of increasing concentrations of the polyamine antagonist, arcaine, on Glu Con-G (SEQ ID NO:5) enhanced [³H]MK-801 binding to well washed brain membranes.

Figure 15 shows the effect of increasing concentrations of the polyamine antagonist, arcaine, on Glu Con-G (12-17) (SEQ ID NO: 7) enhanced [³H]MK-801 binding to well washed brain membranes.

Figure 16 shows Table I which summarizes in terms of potency and efficacy the agonist and partial agonist activities of polyamines, Mg⁺⁺, and the Con-G derivatives. Higher potencies are reflected in smaller $EC_{50}$ values while higher efficacies are reflected in larger efficacy values.

Figure 17 shows the neuroprotective effect, as percentage protection, of MK-801 and Con-G (SEQ ID NO: 1) on glutamate (100 μM) induced cell death (neurotoxicity) in cerebellar granule cell cultures.

## Detailed Description of the Invention And Best Mode for Carrying Out the Invention

This detailed description is presented in three parts. First, several terms frequently used in this description are defined, some of them because their meanings are not yet standardized in the literature. Second, a brief description of the materials and methods which were used to synthesize and measure the action of the Con-G class of compounds is presented. Third, so that those skilled in the art may more fully understand and use the invention, a summary of the actions of the various compounds of the invention is presented along with a discussion of the chemical/structural requirements for activity.

Glossary of Terms

The following terms are defined so that their use in this application is unambiguous:

The term **"agonist"** as used herein means any compound which increases the flow of $Ca^{++}$ through the NMDA receptor - a channel opener.

The term **"antagonist"** as used herein means any compound which reduces the flow of $Ca^{++}$ through the NMDA receptor - a channel closer.

The abbreviation **"Con-G"** as used herein means Conantokin-G.

The abbreviation **"Con-T"** as used herein means Conantokin-T.

The abbreviation **"D-Glu"** as used herein means the amino acid D-glutamate.

The term **"functional antagonist"** as used herein means a compound which reduces the flow of ions through the NMDA receptor ligand-gated cation channel. A **functional antagonist** partially closes the NMDA receptor channel. **Functional antagonists** include compounds possessing partial agonist properties for allosteric modulatory sites on the NMDA receptor including a polyamine associated modulatory site.

The abbreviation **"Gla"** as used herein means the amino acid $\gamma$-carboxyglutamate.

The abbreviation **"Glu"** as used herein means the amino acid L-glutamate.

The term **"neuropsychopharmacological disorder"** as used herein means a disorder resulting from or associated with a reduced or excessive flux of ions through the NMDA receptor ligand-gated cation channel.

The term **"NMDA receptor"** as used herein means a postsynaptic receptor which is stimulated, at a minimum, by the excitatory amino acids glutamate and glycine. It is a ligand-gated receptor with a strychnine insensitive glycine modulatory site.

The term **"partial agonist"** as used herein means a compound which modulates an allosteric site on the NMDA receptor so as to increase or decrease the flux of $Ca^{++}$ through the ligand-gated channel depending on the presence or absence of the principal site modulator(s). In the absence of the principal site modulator(s), a **partial agonist** increases the flow of $Ca^{++}$ through the ligand-gated channel but at a lower flux than achieved by the principal site modulator(s). A **partial agonist** partially opens the NMDA receptor channel. In the presence of the principal site modulator(s), a **partial agonist** decreases the flow of $Ca^{++}$ through the ligand-gated channel below the flux normally achieved by the principal site modulator(s). Therefore, in the presence of the principal site modulator(s), a **partial agonist** is also one type of **functional antagonist.**

Methods and Materials:

Membrane Preparation: Male Sprague-Dawley rats (175-300 g., Taconic Farms, Germantown, NY) were killed by decapitation. Whole brains (minus cerebella) were removed and homogenized with a Polytron homogenizer (setting 6, 30s.) using 10 volumes of 0.32 M sucrose in the assay buffer (5mM Hepes/4.5 mM Tris buffer, pH 7.8). The homogenate was diluted to 50 volumes with assay buffer and centrifuged at 1,000 x g for 10 minutes. The supernatant was decanted and recentrifuged at 20,000 x g for 20 minutes. The resulting pellet was resuspended in 50 volumes of assay buffer and centrifuged at 8,000 x g for 20 minutes. The supernatant and outer "buffy" pellet coat were collected and centrifuged at 20,000 x g for 20 minutes. The supernatant was discarded, and the pellet was resuspended in 50 volumes of assay buffer containing 1 MM EDTA prior to recentrifugation at 20,000 x g for 20 minutes. This resuspension/centrifugation procedure was repeated three times. The last two cycles were performed using assay buffer without EDTA. The resulting pellet was resuspended in 5 volumes of assay buffer, frozen over solid $CO_2$, and stored at -70°C. On the day of assay the tissue was thawed, diluted to 50 volumes with assay buffer, and centrifuged at 20,000 x g for 20 minutes. The supernatant was discarded, and the resulting pellet was resuspended in 50 volumes of assay buffer and centrifuged at 20,000 x g for 20 minutes. The final pellet was resuspended in 30 volumes of assay buffer without further modification.

Peptide Synthesis: Con-G (SEQ ID NO: 1) was synthesized and purified by a modification of the method of Rivier et al. (1987). Synthesis of the Tyr0-Conantokin (Tyr0 Con-G (SEQ ID NO: 3)) analog was accomplished by an additional carbodiimide-mediated coupling step with FMOC-Tyr (O-tbu)-OH to the final peptide resin. The $N^{\alpha}$-acetyl Con-G (NAc Con-G (SEQ ID NO: 4)) analog was prepared by treating the peptide resin with 20% piperidine in DMF for 30 minutes to remove the FMOC group. Following a series of washes with DMF, dichloromethane, and isopropyl alcohol, a positive Kaiser test (Kaiser et al., 1970) was obtained indicating complete removal of the FMOC group. The peptide-resin was treated with 20% acetic anhydride in DMF for 30 minutes. A negative Kaiser test indicated complete blocking of the N-terminal amino group. All other analogs were synthesized using a BOC/Bzl strategy incorporating the preprogrammed protocols of an automated peptide synthesizer (Applied Biosystems 431-A, Foster City, CA). Cleavage and deprotection of each of the analogs was accomplished by treatment with liquid HF in the presence of m-cresol according to the method of Sakakibara et al. (1967). Each analog was purified to homogeneity by reverse-phase HPLC on a Waters $C_{18}$ silicon column. Each analog was purified using a linear gradient from 10% to 30% acetonitrile into $H_2O$ containing 0.1% TFA over 60 minutes. Highly pure fractions were combined and lyophilized. Amino acid analysis results were within 5% of theoretical for each peptide.

Radioligand Binding: Assays were performed in a total volume of 500μl containing ~200 μg protein, [3H]MK-801 (final concentration, 4-5 nM), and test compounds or buffer. Assays were incubated for 2 hours at room temperature and terminated by rapid filtration under partial vacuum (Brandel Cell Harvester, Model M-24R) with two 5 ml washes of buffer over glass fiber filters that were presoaked in 0.03% polyethyleneimine. Nonspecific binding was determined using PCP (100 μM) and represented ~15-50% of the total binding in the absence of modulatory agents.

Preparation of cerebellar granule cells: Primary cultures of cerebellar granule cells were prepared from 6-8 day old Sprague-Dawley rats by the method of Gallo et al. (1982). Dissociated cells were resuspended in Eagle's Basal Media with 10% fetal calf serum, 2 mM 1-glutamine, 0.1 mg/ml gentamicin, and 25 mM KCl. The cells were seeded in 35 mm ply-L-lysine coated dishes at a density of 1.8-2.8 x $10^5$ cells/cm2. Cytosine arabinoside (10 /μM) was added 18 to 24 hours after plating to inhibit the growth of non-neuronal cells. Cultures generated by this method contained > 90% granule cell neurons. Media were unchanged during the culture period.

Evaluation of neurotoxicity: Experiments were performed using cells cultured for 9 days. The culture medium was removed and the granule cells were washed twice with 1.5 ml of incubation buffer (160.6 mM NaCl, 5.6 mM KCl, 2.3 mM $CaCl_2$, and 8.6 mM HEPES, pH 7.4 - Locke buffer without magnesium or glucose). The cells were preincubated in 1 ml of buffer for 25 minutes at 37°C. Following a change of buffer, drugs were added to the appropriate cultures and incubated for an additional 15 minutes (37°C). The buffer was changed again, and glutamate (100 μM) was added together with Con-G (SEQ ID NO: 1) (0.1-5μM), MK-801 (10 μM), or vehicle. Thirty minutes later, the buffer was removed, the cells washed twice with 1.5 ml of complete Locke buffer (154 mM NaCl, 5.6 mM KCl, 2.3 mM $CaCl_2$, 5.6 mM glucose, 8.6 mM HEPES, 1.0 mM $MgCl_2$, pH 7.4), and the cells incubated for 18-24 hours in 1 ml of culture medium. Cell death was determined using trypan blue exclusion. Cell death and neuroprotection were calculated by the following equations:

$$\% \text{ cytotoxicity} = D/(D+L) \times 100; \% \text{ protection} = 100 - (\%$$

$$\text{cytotoxicity})$$

where D equals the number of dead cells counted in three different random fields using 400x bright field microscopy, and L equals the number of live cells counted in the same three fields.

Materials: Amino acid derivatives and solid-phase resins were obtained from Bachem Feinchemikalien AG (Bubendorf, Switzerland) and from Dr. Robert Williams (Colorado State University). [3H]MK-801 (Sp. Act. 28.8 Ci/mmol) was obtained from DuPont-NEN (Boston, MA). Eagle's Basal Media was purchased from GIBCO (Grand Island, NY). Fetal calf serum was from Quality Biological, Inc. (Gaithersburg, MD). Other cell culture reagents and spermine were purchased from Sigma Co. (St. Louis, MO). All other materials were obtained from standard commercial sources.

Description Of Allosteric Modulatory Behavior Of Inventive Compounds

While Mena et al. (1990) had reported that Con-G (SEQ ID NO: 1) was an NMDA receptor antagonist which was not competitive with previously known agonists or antagonists, no one had: 1) elucidated the underlying mechanism by which the antagonism is mediated; 2) taught how such antagonism can be utilized to control properties of the receptor; or 3) described accurately the chemical/biological structural requirements for modulatory activity. It has been discovered that Con-G (SEQ ID NO: 1) inhibits the stimulatory effect of polyamines on the NMDA receptor. However, Con-G (SEQ ID NO: 1) does not act directly at the polyamine site but appears to regulate the response of the polyamine site by action at a new and unique modulatory site.

Under non-equilibrium conditions [3H]MK-801 binding to the NMDA receptor has been shown to be a sensitive and specific measure of the opening of the NMDA ligand-gated cation channel. Since [3H]MK-801 binds to the inside of the cation channel in the NMDA receptor, increased [3H]MK-801 binding reflects an increase in the open state of the channel.

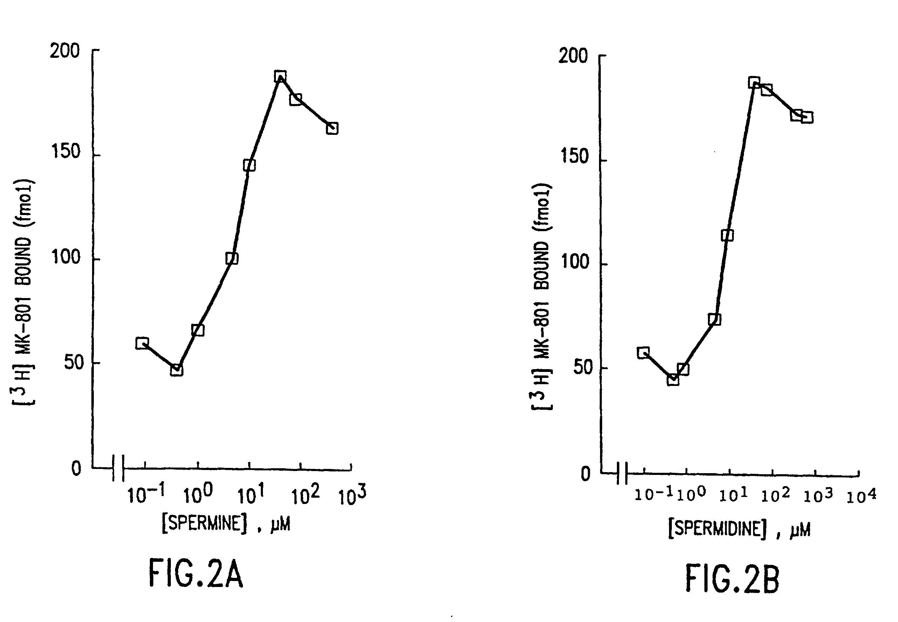

Figure 2 shows a plot of the binding of [3H]MK-801 as a function of increasing concentrations of spermine, Figure 2(A), and spermidine, Figure 2(B), in the nominal absence of endogenous glutamate and glycine. (While the forebrain membrane preparations were very well washed, as noted in the methods section above, it is believed that it is not entirely possible to totally remove these amino acids and that some remain in all these assays.) It can be seen that both of these polyamines stimulates the binding of [3H]MK-801 in a concentration dependent fashion. Clearly the polyamines allosterically modulate the opening of the ligand-gated NMDA channel.

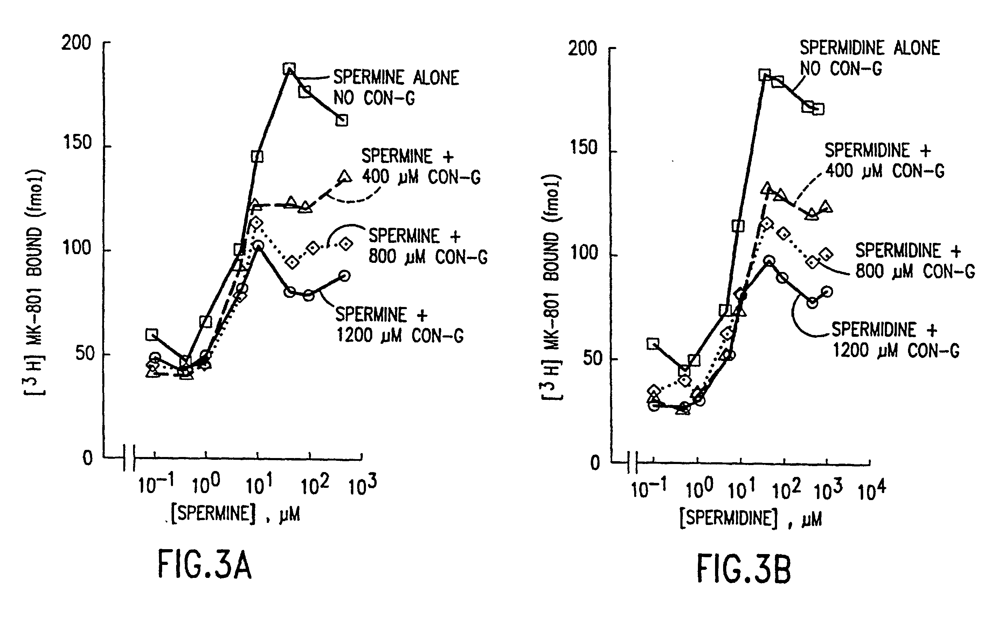

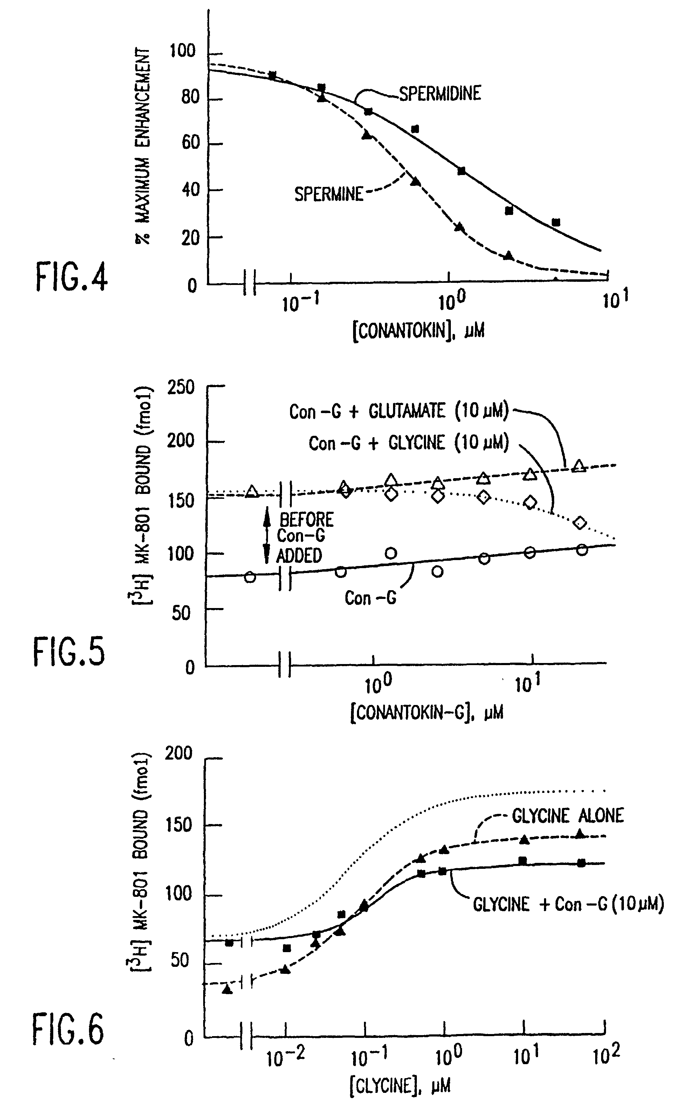

Figure 3 shows the same plots as in Figure 2 with the addition of curves representing the [3H]MK-801 binding observed when varying amounts of Con-G (SEQ ID NO: 1) are added to the membrane preparation. It can be seen that as the concentration of Con-G (SEQ ID NO: 1) increases from 400 nM to 1200 nM, the degree of polyamine stimulated [3H]MK-801 binding decreases reflecting a more and more closed state of the channel. Con-G (SEQ ID NO: 1) does not decrease the polyamine stimulated binding in a concentration dependant manner consistent with competitive inhibition but does decrease the stimulation in a manner consistent with non-competitive inhibition of the polyamines. Figure 4 shows a plot of the reduction in maximum stimulation of [3H]MK-801 binding achieved by spermine and spermidine as the concentration of Con-G (SEQ ID NO: 1) increases. There is no consistent relationship between the degree of inhibition of the polyamine stimulation and the concentration of Con-G (SEQ ID NO: 1) such as is found with the polyamines diethylenetriamine and arcaine which inhibit spermine and spermidine enhanced [3H]MK-801 binding through a competitive antagonism at the polyamine site. Thus, from these examples it is clear that Con-G (SEQ ID NO: 1) is not acting as a competitive inhibitor of the polyamines at the polyamine site.

Figure 5 shows the effect of increasing concentrations of Con-G (SEQ ID NO: 1) upon basal [3H]MK-801 binding and glycine and glutamate stimulated [3H]MK-801 binding to the brain membrane preparation. Con-G (SEQ ID NO: 1) produces a small increase in basal [3H]MK-801 binding which is very modest compared to the enhanced binding produced by glutamate, glycine, and the polyamines. Con-G (SEQ ID NO: 1) has no affect upon glutamate stimulation and the glutamate stimulation is additive with that produced by Con-G (SEQ ID NO: 1) alone. Con-G (SEQ ID NO: 1) does slightly inhibit glycine stimulation at concentrations greater than 5μM. However, no effect on glycine's potency is seen. Figure 6 shows the effect of 10 μM Con-G (SEQ ID NO: 1) on glycine stimulated [3H]MK-801 binding to the membrane preparation. It can be seen that Con-G (SEQ ID NO: 1) does not inhibit the glycine stimulation in a manner consistent with competitive inhibition. Therefore, Con-G (SEQ ID NO: 1) does not appear to be a competitive inhibitor of the glycine site. Further, there is some evidence that has been reported suggesting that the polyamines increase receptor response by increasing the apparent affinity of glycine at the strychnine-insensitive glycine site. It is, therefore, possible that the modest reduction of glycine stimulation is attributable to Con-G (SEQ ID NO: 1) inhibition of some remaining endogenous polyamines in the washed brain membranes. Thus, Con-G (SEQ ID NO: 1) also does not appear to act at the glutamate or glycine binding sites.

Ifenprodil also inhibits polyamine binding in a non-competitive manner, and it might have been possible that Con-G (SEQ ID NO: 1) acts at the same site as ifenprodil. However, Con-G (SEQ ID NO: 1) does not seem to act at the same site as ifenprodil since Con-G (SEQ ID NO: 1) slightly increases, while ifenprodil decreases (Reynolds and Miller, 1989), [3H]MK-801 binding in the nominal absence of glycine and glutamate. Further, ifenprodil inhibits glutamate enhanced [3H]MK-801 binding, while as shown in Figure 5, even very high concentrations of Con-G (SEQ ID NO: 1) do not appear to affect glutamate stimulation.

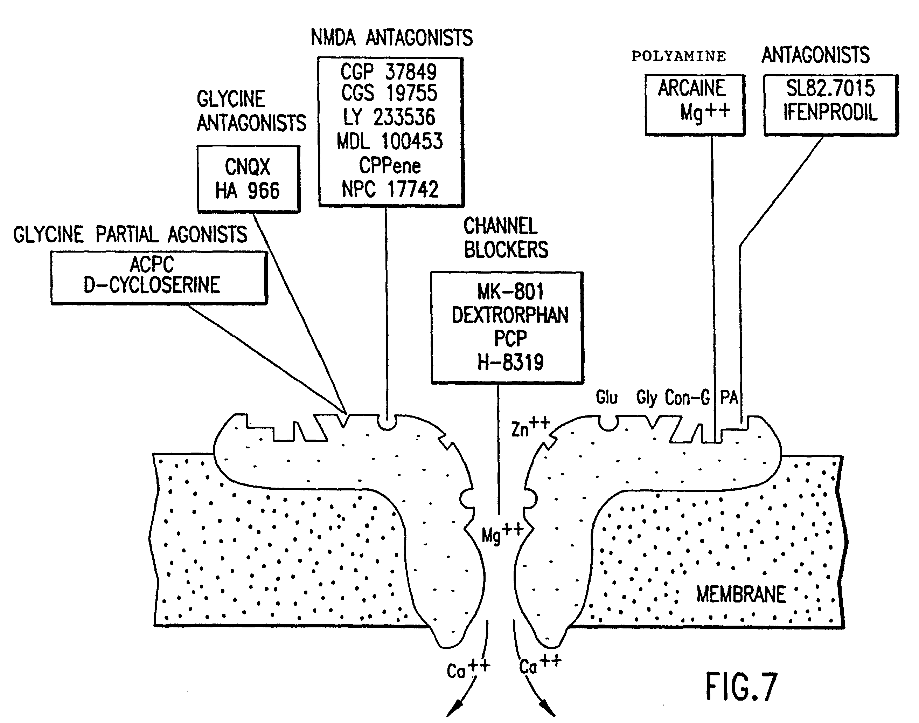

In summary, from the above it is clear that Con-G (SEQ ID NO: 1) must be acting at a novel and previously unrecognized allosteric modulatory/regulatory site on the NMDA receptor. In Figure 7 this site is labeled Con-G. For purposes of indicating that Con-G (SEQ ID NO: 1) affects polyamine stimulation, the Con-G (SEQ ID NO: 1) site it has been placed adjacent to the polyamine site. Such placement is not meant to indicate the actual site arrangement on the receptor. Similarly, the site of action of ifenprodil is placed adjacent to the polyamine site. The discovery that Con-G (SEQ ID NO: 1) acts as a non-competitive antagonist of polyamine stimulation of the NMDA receptor indicates that it may be pharmaceutically used to reduce/modify the NMDA receptor response both in those instances where the enhanced $Ca^{++}$ flux through the NMDA receptor is stimulated by polyamines and in those situations where excess stimulation by other allosteric modulators relies on an appropriate level of polyamines being present and active.

The ability of Con-G (SEQ ID NO: 1) to act as a strong non-competitive NMDA antagonist at the newly identified modulatory site is abolished by certain modifications of the Con-G (SEQ ID NO: 1) peptide structure. In fact, it has been discovered that modification of either the N- terminus or replacement of the Gla residues by Glu produces several derivatives which may act as partial agonists (positive allosteric modulators) with polyamine-like profiles. For illustration, the ten derivatives set out below will be discussed. It should be under-stood, however, that these are representative compounds which are sufficient to teach the underlying principles of the invention relating to structure/activity relationships to persons skilled in the art and, therefore, are not limiting of this disclosure. The numbers above the sequences indicate each peptide's position based on the parent Con-G (SEQ ID NO: 1) molecule. γ-carboxyglutamates, Gla, are indicated by boldface. In those places where glutamate, Glu, was substituted for γ-carboxyglutamate, Glu has been italicized. The nomenclature/abbreviation used to identify each compound throughout this patent and in the claims is set out to the left beside the corresponding polypeptide structure.

| | 0 | 1 | 2 | 3 | 4 | 5 | 6 | 7 | 8 | 9 | 10 | 11 | 12 | 13 | 14 | 15 | 16 | 17 | |
|---|---|---|---|---|---|---|---|---|---|---|---|---|---|---|---|---|---|---|---|
| Con-G: | | Gly | Glu | Gla | Gla | Leu | Gln | Gla | Asn | Gln | Gla | Leu | Ile | Arg | Gla | Lys | Ser | Asn-NH₂ | SEQ ID NO: 1 |
| Con-G OH: | | Gly | Glu | Gla | Gla | Leu | Gln | Gla | Asn | Gln | Gla | Leu | Ile | Arg | Gla | Lys | Ser | Asn-*OH* | SEQ ID NO: 2 |
| Tyr⁰ Con-G: | *Tyr* | Gly | Glu | Gla | Gla | Leu | Gln | Gla | Asn | Gln | Gla | Leu | Ile | Arg | Gla | Lys | Ser | Asn-NH₂ | SEQ ID NO: 3 |
| NAc Con-G: | *Ac* | Gly | Glu | Gla | Gla | Leu | Gln | Gla | Asn | Gln | Gla | Leu | Ile | Arg | Gla | Lys | Ser | Asn-NH₂ | SEQ ID NO: 4 |
| Glu Con-G: | | Gly | Glu | *Glu* | *Glu* | Leu | Gln | *Glu* | Asn | Gln | *Glu* | Leu | Ile | Arg | *Glu* | Lys | Ser | Asn-NH₂ | SEQ ID NO: 5 |
| Tyr⁰ Glu Con-G: | *Tyr* | Gly | Glu | *Glu* | *Glu* | Leu | Gln | *Glu* | Asn | Gln | *Glu* | Leu | Ile | Arg | *Glu* | Lys | Ser | Asn-NH₂ | SEQ ID NO: 6 |
| Glu Con-G(12-17): | | | | | | | | | | | | | Ile | Arg | *Glu* | Lys | Ser | Asn-NH₂ | SEQ ID NO: 7 |
| Glu Con-G(9-17): | | | | | | | | | | Gln | *Glu* | Leu | Ile | Arg | *Glu* | Lys | Ser | Asn-NH₂ | SEQ ID NO: 8 |
| Glu Con-G(6-17): | | | | | | | Gln | *Glu* | Asn | Gln | *Glu* | Leu | Ile | Arg | *Glu* | Lys | Ser | Asn-NH₂ | SEQ ID NO: 9 |
| Glu Con-G(2-17): | | | Glu | *Glu* | *Glu* | Leu | Gln | *Glu* | Asn | Gln | *Glu* | Leu | Ile | Arg | *Glu* | Lys | Ser | Asn-NH₂ | SEQ ID NO: 10 |
| D-Glu Con-G: | | Gly | Glu | *DGlu* | *DGlu* | Leu | Gln | *DGlu* | Asn | Gln | *DGlu* | Leu | Ile | Arg | *DGlu* | Lys | Ser | Asn-NH₂ | SEQ ID NO: 11 |

In Con-G OH (SEQ ID NO: 2) the amide group on the carboxyl terminal peptide, Asparagine, is replaced with a hydroxy group. Tyr⁰ Con-G (SEQ ID NO: 3) adds one amino acid to the Con-G (SEQ ID NO: 1) molecule at the N-

terminus while Tyr[0] Glu Con-G (SEQ ID NO: 6) adds the same amino acid to the N- terminus of the glutamate substituted Con-G. NAc Con-G (SEQ ID NO: 4) places an acetyl group on the N- terminus peptide of Con-G (SEQ ID NO: 1). Glu Con-G (SEQ ID NO:5) is formed by the substitution of L-glutamate for the γ-carboxyglutamate (Gla) which is found in the native Con-G. Several fragments of the Glu Con-G (SEQ ID NO:5) derivative which keep the carboxyl terminus of Glu Con-G (SEQ ID NO:5) intact are shown. Each fragment is identified by the amino acid positions of the Glu Con-G (SEQ ID NO:5) sequence which remain in the fragment. Finally, D-Glu Con-G (SEQ ID NO: 11) is formed by the substitution of D-glutamate for the γ-carboxyglutamate (Gla) which is found in the native Con-G (SEQ ID NO: 1).

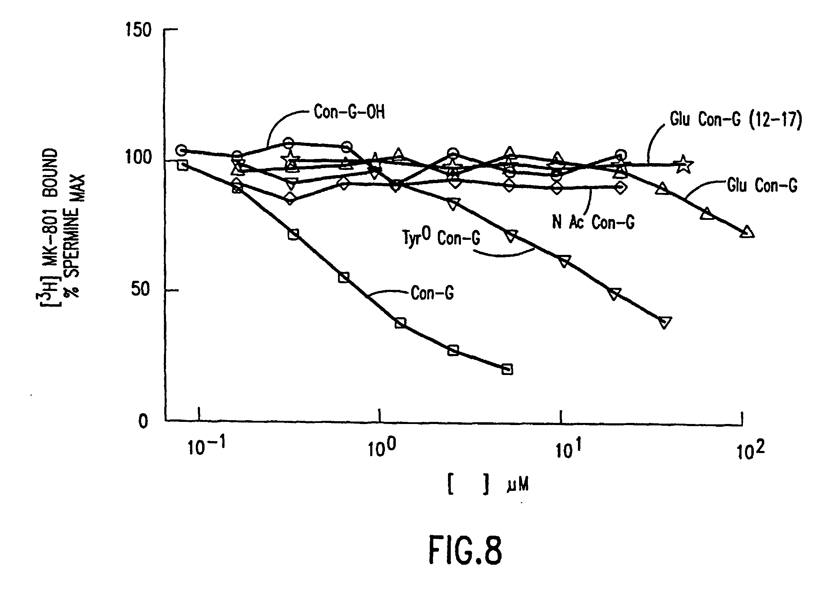

Figure 8 shows the effect of increasing concentrations of Con-G (SEQ ID NO: 1), Glu Con-G (SEQ ID NO: 5), Con-G OH (SEQ ID NO: 2), NAc Con-G (SEQ ID NO: 4), Tyr[0] Con-G (SEQ ID NO: 3), and Glu Con-G(12-17) (SEQ ID NO: 7) on the maximum spermine induced enhancement of [3H]MK-801 binding to the NMDA receptor in well washed brain membranes. As shown earlier in Figure 3, Con-G (SEQ ID NO: 1) acts as a very strong antagonist. However, Con-G OH (SEQ ID NO: 2), NAc Con-G (SEQ ID NO: 4), and Glu Con-G(12-17) (SEQ ID NO: 7) have substantially no effect on polyamine stimulated [3H]MK-801 binding. Tyr[0] Con-G (SEQ ID NO: 3) and Glu Con-G (SEQ ID NO:5) have substantially no effect on polyamine stimulated [3H]MK-801 binding up to concentrations well beyond those required for effective antagonism by Con-G (SEQ ID NO: 1). Tyr[0] Con-G (SEQ ID NO: 3) exhibits a significantly reduced inhibition of [3H]MK-801 binding compared to native Con-G (SEQ ID NO: 1) requiring approximately an order of magnitude greater concentration to show the same decrease. Glu Con-G (SEQ ID NO:5) does not begin to show any antagonism until its concentration is over an order of magnitude higher than the concentration at which Tyr[0] Con-G (SEQ ID NO: 3) begins to show antagonism. Thus, modification at either end of the Con-G (SEQ ID NO: 1) polypeptide or replacement of the γ-carboxyglutamate by glutamate is sufficient to abolish or substantially reduce the noncompetitive inhibition of the polyamine response characteristic of the parent Con-G (SEQ ID NO: 1) polypeptide.

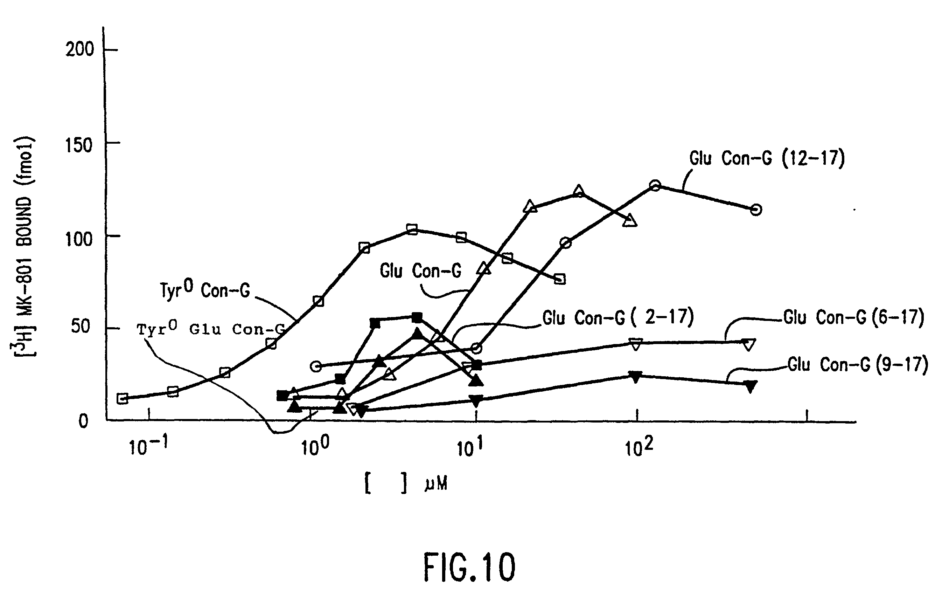

Figure 9 shows the effect of Tyr[0] Con-G (SEQ ID NO: 3) and Glu Con-G (SEQ ID NO: 5) on [3H]MK-801 binding in the presence and absence of the polyamine spermine. As can be seen from Figure 8 and the top two curves of Figure 9, at higher concentrations Glu Con-G (SEQ ID NO:5) and Tyr[0] Con-G (SEQ ID NO: 3) exhibit a functional antagonism (down modulation) to polyamine enhanced [3H]MK-801 binding. However, the levels of such antagonism are nowhere near those characteristic of the native Con-G (SEQ ID NO: 1). Remarkably, as shown in the lower two curves of Figure 9, in the absence of polyamine, both Glu Con-G (SEQ ID NO:5) and Tyr[0] Con-G (SEQ ID NO: 3) show a concentration dependent enhancement (up modulation) of [3H]MK-801 binding. This antagonism/agonism dual behavior is typical of the type of compound identified as a "partial agonist". In addition to Glu Con-G (SEQ ID NO:5) and Try[0] Con-G several other derivatives of Con-G (SEQ ID NO: 1) show similar concentration dependant enhancements of [3H]MK-801 binding in the absence of polyamines as can be seen in Figure 10 for Glu Con-G (12-17) (SEQ ID NO: 7), Glu Con-G (2-17) (SEQ ID NO: 10). Tyr[0] Glu Con-G (SEQ ID NO: 6), Glu Con-G (6-17) (SEQ ID NO: 9), and Glu Con-G (9-17) (SEQ ID NO: 8).

Figure 11(A) shows the maximum NMDA receptor stimulation (enhancement of [3H]MK-801 binding) achieved with Glu Con-G(12-17) (SEQ ID NO: 7), Glu Con-G (SEQ ID NO: 5), Tyr[0] Con-G (SEQ ID NO: 3), Glu Con-G(2-17) (SEQ ID NO: 10), D-Glu Con-G (SEQ ID NO: 11), Tyr° Glu Con-G (SEQ ID NO: 6), and Glu Con-G(9-17) (SEQ ID NO: 8) as a percentage of the maximum enhancement produced by the polyamine spermine. Glu Con-G(12-17) (SEQ ID NO: 7), Glu Con-G (SEQ ID NO: 5), and Tyr[0] Con-G (SEQ ID NO: 3) achieve about 70% agonist activity when compared to spermine. Glu Con-G(2-17) (SEQ ID NO: 10), D-Glu Con-G (SEQ ID NO: 11), and Tyr[0] Glu Con-G (SEQ ID NO: 6) achieve about 40% agonist activity compared to spermine. While not shown in Figures 10 or 11, NAc Con-G (SEQ ID NO: 4) produces a relatively minimal stimulation similar to that of Glu Con-G(6-17) (SEQ ID NO: 9) and Con-G OH (SEQ ID NO: 2) does not produce any stimulation of [3H]MK-801 binding in the absence of polyamines.

The potencies of Glu Con-G (12-17) (SEQ ID NO: 7), Spermine, D-Glu Con-G (SEQ ID NO: 11), Glu Con-G (SEQ ID NO: 5), Tyr[0] Glu Con-G (SEQ ID NO: 6), Tyr° Con-G (SEQ ID NO: 3), and Glu Con-G (2-17) (SEQ ID NO: 10) are shown in Figure 11(B). While Glu Con-G (SEQ ID NO:5) and D-Glu Con-G (SEQ ID NO: 11) exhibited potencies about the same as spermine, Tyr[0] Con-G (SEQ ID NO: 3) and Glu Con-G(2-17) (SEQ ID NO: 10), although less efficacious than spermine, show potencies about 7 times as great as spermine. Thus, Tyr[0] Con-G (SEQ ID NO: 3) and Glu Con-G(2-17) (SEQ ID NO: 10) exhibit potencies for modulation of a polyamine associated site about 7 times greater than any previously known modulator of the polyamine site. On the other hand, Glu Con-G(12-17) (SEQ ID NO: 7), while about 70% as efficacious as spermine, exhibits a potency approximately 6 times less than spermine.

The agonist effect of Glu Con-G(12-17) (SEQ ID NO: 7) is remarkable. Glu Con-G(12-17) (SEQ ID NO: 7) has been discovered to be the shortest, high efficacy, agonist of the polyamine associated site yet known. Since Glu Con-G(12-17) (SEQ ID NO: 7) retains only the carboxyl terminus of the Glu Con-G (SEQ ID NO:5) polypeptide, it is clear that it is the carboxyl terminus which is critical to the stimulatory effect of the Con-G derivatives. This is consistent with the observation that Con-G OH (SEQ ID NO: 2) produces no stimulatory effect.

Figure 9 clearly shows that Glu Con-G (SEQ ID NO:5) and Tyr[0] Con-G (SEQ ID NO: 3) inhibit spermine stimulated [3H]MK-801 binding to substantially the same levels as Glu Con-G (SEQ ID NO:5) and Tyr[0] Con-G (SEQ ID NO: 3) stimulate [3H]MK-801 binding in the absence of spermine. Such behavior is characteristic of partial agonists.

In fact, the effects of the Con-G derivatives can be viewed from two different perspectives. First, in the presence of polyamines the derivatives can be viewed as functional antagonists. Second, in the absence of polyamines, the derivatives can be viewed as partial agonists.

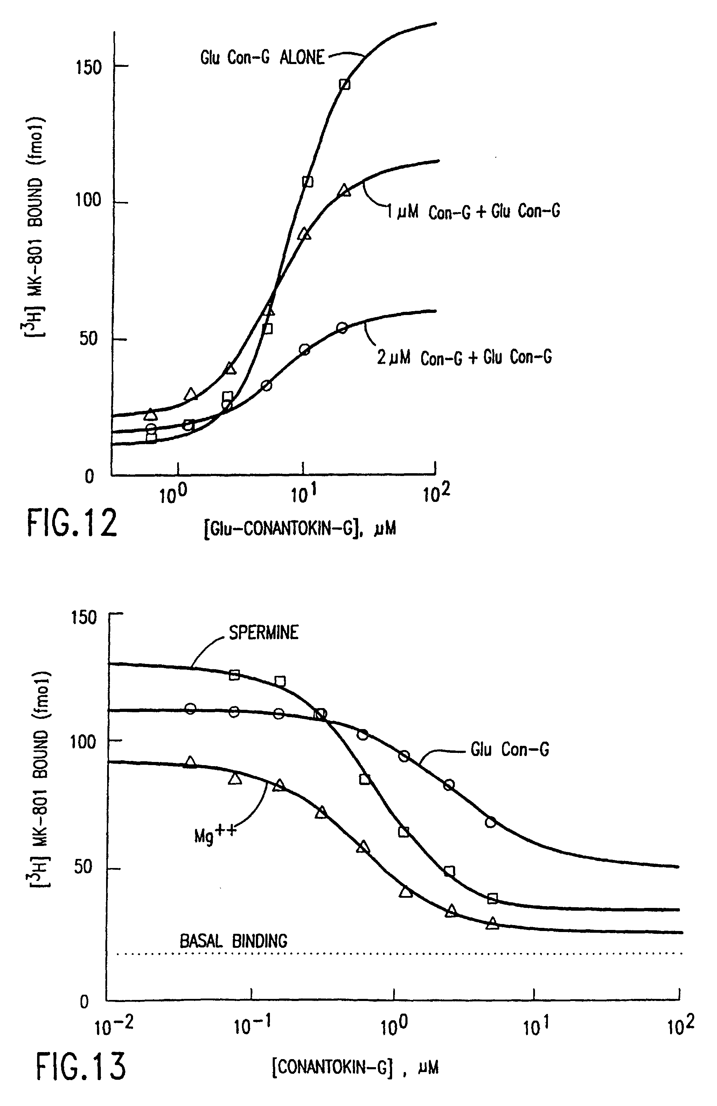

Whether the activity of the Con-G derivatives as partial agonists reflects modulation of the polyamine site directly is not entirely clear at the present time. The fact that they exhibit polyamine-like agonistic properties suggests that they may be acting at the polyamine site. As shown in Figure 12, supporting this possibility is the fact that Con-G (SEQ ID NO: 1), which has been discovered to inhibit polyamine modulation of the polyamine site, also inhibits Glu Con-G (SEQ ID NO:5) stimulated [$^3$H]MK-801 binding, thus suggesting that Glu Con-G (SEQ ID NO:5) also acts at the polyamine site. Figure 13 shows the concentration dependent inhibition of Glu Con-G (SEQ ID NO: 5), spermine, and Mg$^{++}$ enhanced [$^3$H]MK-801 binding by Con-G (SEQ ID NO: 1). While the inhibition by Con-G (SEQ ID NO: 1) of Glu Con-G (SEQ ID NO:5) stimulated binding is concentration dependent, the inhibition is not altered in a manner as would be expected of a competitive inhibitor. Therefore, Con-G (SEQ ID NO: 1) does not competitively inhibit Glu Con-G (SEQ ID NO:5) stimulation as it does not competitively inhibit polyamine stimulation. Since Con-G (SEQ ID NO: 1) acts as an antagonist to the polyamine site, antagonism to Con-G derivative stimulation suggests that the Con-G derivatives bind to the polyamine site.

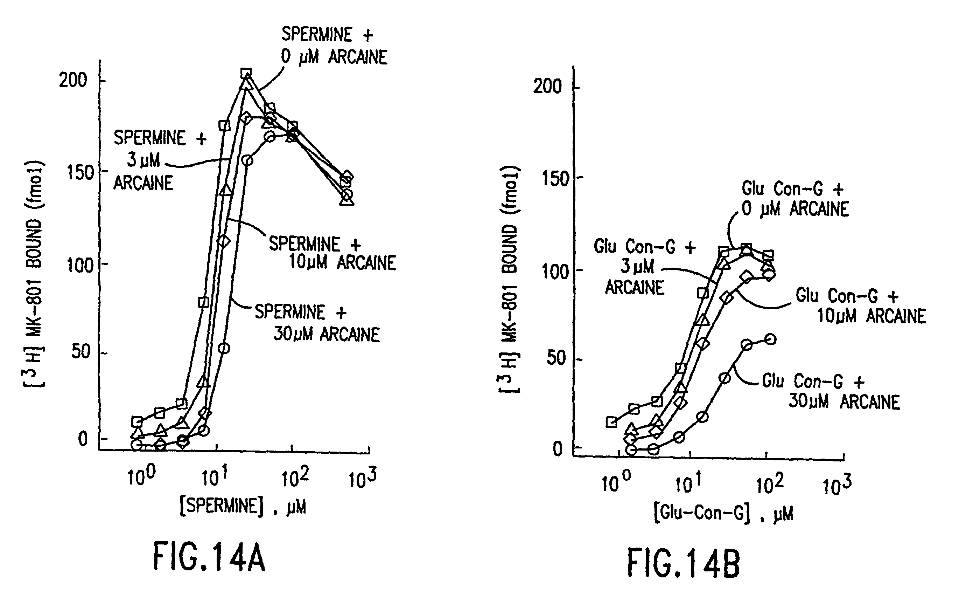

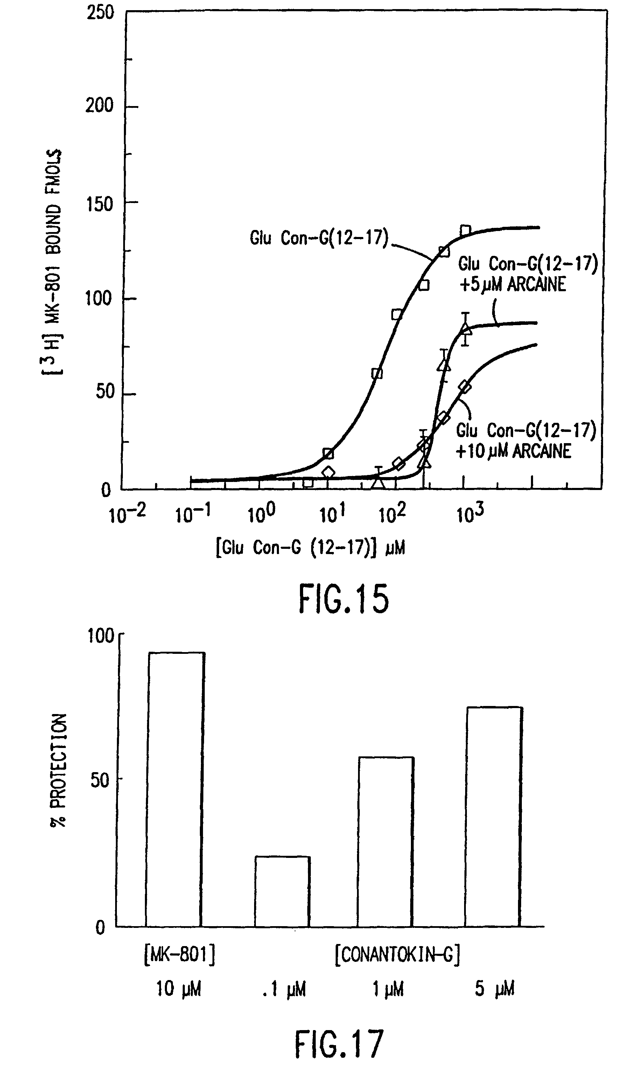

Further evidence that the Con-G derivatives act at the polyamine site is given by their interactions with a competitive inhibitor of the polyamine site. Figure 14(A) shows the effect of increasing concentrations of arcaine on spermine stimulation of [$^3$H]MK-801 binding. Figure 14(B) shows the effect of increasing concentrations of arcaine on the Glu Con-G (SEQ ID NO:5) stimulation of [$^3$H]MK-801 binding. Similarly, Figure 15 shows the shows the effect of increasing concentrations of arcaine on the Glu Con-G(12-17) (SEQ ID NO: 7) stimulation of [$^3$H]MK-801 binding. Since arcaine is considered a competitive polyamine antagonist at the polyamine site, as shown by its activity at increasing concentrations in Figure 14(A), and since arcaine appears to inhibit Glu Con-G (SEQ ID NO: 5) and Glu Con-G(12-17) (SEQ ID NO: 7) stimulation in the same manner as it inhibits spermine stimulation, the Con-G derivatives may well be acting at the polyamine site. However, since the site is not unambiguously identified, and in consideration of possible structural requirements discussed below, the allosteric modulatory site at which the Con-G derivatives affect their action is referred to throughout this application as a "polyamine associated site".

From circular dichroism and other studies Con-G (SEQ ID NO: 1) is believed to exist as an α-helix. A "helical wheel" projection of the Con-G (SEQ ID NO: 1) peptide sequence places all but one of the doubly charged Gla residues substantially along one side of the helix. It has also been noted that the placement of the Gla residues along the peptide chain is highly conserved between Con-G (SEQ ID NO: 1) and Con-T (SEQ ID NO: 12). Thus, it has been postulated by others that the alignment of the charged Gla residues as achieved through the α-helix is necessary and sufficient for Con-G's activity at the NMDA receptor. It has been discovered by studying the comparative actions of the Con-G derivatives disclosed above that the chemical/structural requirements both for the antagonistic activity of Con-G (SEQ ID NO: 1) and for the partial agonist modulation of a polyamine associated site are much more complex.

For instance, Con-G OH (SEQ ID NO: 2) shows no activity, either inhibitory or stimulatory, yet the Con-G α-helix with its aligned charged Gla residues is still present. Thus, an amidated carboxyl end is necessary to Con-G (SEQ ID NO: 1) antagonist activity. Further, modifications of the N-terminus of Con-G (SEQ ID NO: 1) also change its behavior. Either acylation (a mechanism known to occur naturally to stabilize polypeptides) or the addition of the amino acid Tyr to the N- terminal residue dramatically modify Con-G's activity. NAc Con-G (SEQ ID NO: 4) displays no apparent antagonistic activity and only marginal stimulatory efficacy although the entire polypeptide sequence responsible for Con-G's *a*-helix is still present. The addition of Tyr changes Con-G (SEQ ID NO: 1) from a strong non-competitive antagonist of the polyamine modulatory site to a partial agonist of a polyamine associated site, once again despite the fact that the Con-G (SEQ ID NO: 1) α-helix with its aligned Gla residues remains. Thus, an unmodified N- terminus with a charged -NH$_3$$^+$ is also necessary to Con-G (SEQ ID NO: 1) antagonist activity.

The substitution of Glu for Gla reduces the charges at each of the substituted sites along the side of the α-helix while preserving the helix. This substitution also changes Con-G (SEQ ID NO: 1) from a strong NMDA antagonist modulating a unique Con-G (SEQ ID NO: 1) site to a partial agonist of a polyamine associated site. Further, disruption of the α-helical backbone and relative rotation of the amino acids with respect to each other, as occurs in D-Glu Con-G (SEQ ID NO: 11), again changes the activity of the Con-G (SEQ ID NO: 1) molecule from that of a strong antagonist to a partial agonist. D-Glu Con-G (SEQ ID NO: 11) is not even as efficacious a partial agonist as is Glu Con-G.

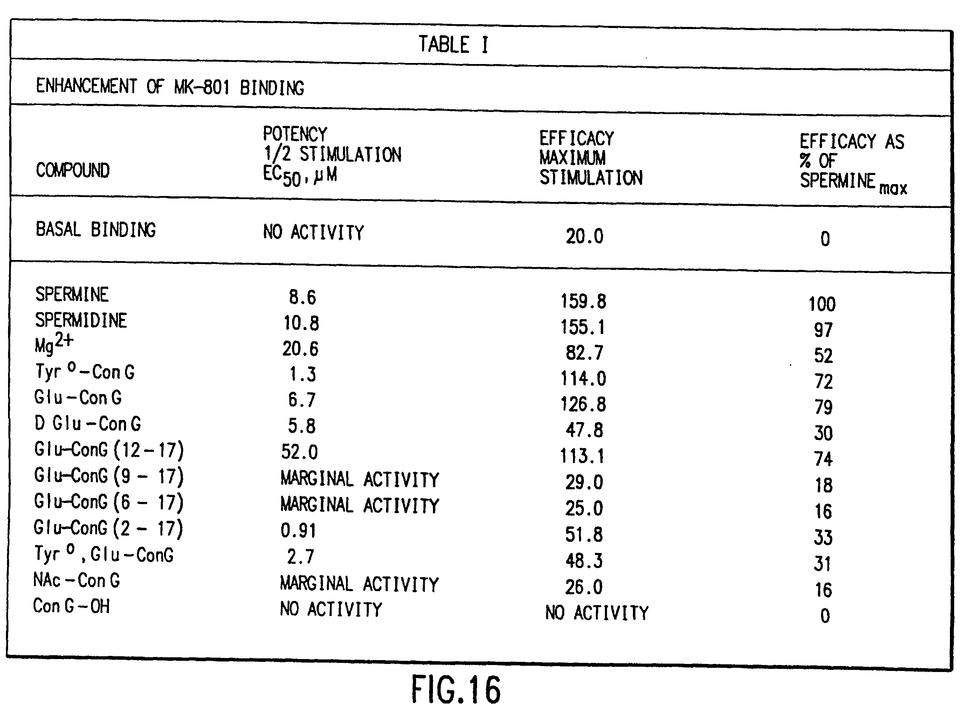

Table I shown in Figure 16 summarizes the effects of the Con-G derivatives in terms of potency and efficacy. Potency is essentially a measure of the affinity of the derivative for the receptor (how tightly the derivative binds). Efficacy is essentially a measure of the stimulatory effect the derivative has on the receptor (how much response it produces). An analysis of the range of activities exhibited by the Con-G derivatives shows that an intact amidated carboxyl terminus is required to achieve high efficacy while the N- terminus structure is crucial to achieving high potency. No where is this dichotomy more evident than in Glu Con-G(12-17) (SEQ ID NO: 7). Polyamine-like agonist activity principally involves the carboxyl terminus. Glu Con-G(12-17) (SEQ ID NO: 7), which preserves the C- terminus, is the shortest and most efficacious polyamine-like agonist yet observed. At the same time, Glu Con-G(12-17) (SEQ ID NO:

EP 0 625 988 B1

7) lacks the necessary N- terminus structure to bind tightly and, in fact, shows one of the lowest potencies observed.

It appears that short derivatives possessing little or no $\alpha$-helical structures but having an intact amidated C-terminus possess polyamine-like agonist activity. As the derivative length approaches the length of the native polypeptide, an amidated C- terminus and increasing percentage of $\alpha$-helicity (due to the longer structure) produces partial agonist (functional antagonist) activity. The presence of helical structure appears to be important for the partial agonist (functional antagonist) function. For Con-G (SEQ ID NO: 1) antagonistic activity, it appears that an amidated carboxyl end, an intact charged ($-NH_3^+$) N- terminus, and properly (charge) aligned $\alpha$-helix are required. Even with an amidated carboxyl end, intact N- terminus, and $\alpha$-helix, modifying the charge distribution along the $\alpha$-helix (as in Glu Con-G) changes the allosteric modulatory properties to that of a partial agonist. Finally, changing Con-G's N- terminus structure by lengthening the Con-G (SEQ ID NO: 1) $\alpha$-helix as in $Tyr^0$ Con-G (SEQ ID NO: 3), although producing a more tightly binding molecule, apparently interferes with the proper alignment or charge distribution necessary for antagonism again producing a partial agonist.

The features of Con-G (SEQ ID NO: 1) and its derivatives noted above are consistent with a physical structure of the NMDA receptor which places the polyamine modulatory site in close proximity to the Con-G (SEQ ID NO: 1) modulatory site. As with all protein structures, although the sites may well be in close physical proximity, they may be separated by many many intervening residues along the folded protein. In such a structure, the N-terminus of Con-G (SEQ ID NO: 1) would bind to part of the Con-G (SEQ ID NO: 1) site while the C- terminus would bind to one part of the polyamine site. A certain length of $\alpha$-helix appears necessary to bridge the distance between the two sites. The highly charged Gla residues along one side of the $\alpha$-helical structure apparently are responsible for holding the molecule in the correct position along the NMDA receptor so that its two ends align properly with their respective sites. The action of Con-G (SEQ ID NO: 1) may well be similar to that of other known ligands which bind in such a manner so as to affect a relative movement of adjoining protein segments towards or away from each other. Such displacement could certainly give rise to the opening and closing of an ion channel. Given this understanding of the structural requirements for Con-G (SEQ ID NO: 1) activity, it is possible to design molecular analogs of Con-G (SEQ ID NO: 1) and its derivatives which possess the required binding characteristics and which are modified to increase or decrease the modulatory effects (activities) at these sites. Thus, not only has the mode of action of Con-G (SEQ ID NO: 1) been elucidated, but also a new class of compounds has been discovered which allosterically modulate a polyamine associated site of the NMDA receptor over a very wide range of activities.

Figure 17 shows that Con-G (SEQ ID NO: 1) may be used to protect cells from excitotoxic death (resulting from excess $Ca^{++}$ influx) induced by glutamate overstimulation of the NMDA receptor. Channel blockers such as MK-801 are known to protect cells from excitotoxic death, but in their case the cure may be as undesirable as the death since the channel blockers block any flux of $Ca^{++}$ thereby eliminating any chance of resumed normal activity. Channel blockers are known to cause hallucinations, high blood pressure, loss of coordination, vacuolarization in the brain, learning disability and memory loss. PCP, for instance, produces a schizophrenic state in man. Figure 17 shows the protective effect of Con-G (SEQ ID NO: 1) and MK-801 on cerebellar granule cell cultures in which glutamate-induced neurotoxicity was measured in response to a 100 $\mu$M concentration of glutamate. Clearly Con-G's action at the Con-G (SEQ ID NO: 1) allosteric modulatory site is sufficient to reduce the flow of $Ca^{++}$ sufficiently to substantially protect the cells from excitotoxic death. In fact, the protection achieved with 5 $\mu$M Con-G (SEQ ID NO: 1) is comparable to that obtained with 10 $\mu$M MK-801. Since Con-G (SEQ ID NO: 1) does not actually block the channel, it should not produce the dramatic long term effects seen with the channel blocking compounds. Again, Con-G's action in allosterically modulating the response of the polyamine site acts to modulate the overall response of the NMDA receptor.

As discussed earlier, Cordi and Trullas have shown that agents which act as partial agonists (functional antagonists) of allosteric modulatory sites on the NMDA receptor may be used to selectively modulate the receptor's function without producing the side effects associated with compounds which block the NMDA channel. In addition, Cordi and Trullas have taught that modulation of NMDA receptor activity by partial agonists (functional antagonists) is useful in the treatment of a wide range of CNS disorders as well as in enhancement of CNS function.

The specific teachings of Cordi and Trullas relate to the use of partial agonists of the glycine site to enhance memory, treat learning and cognitive deficits, treat Alzheimers and age associated memory impairment, treat psychotic disorders, improve memory, and treat mood disorders. The discovery that Con-G (SEQ ID NO: 1) effects the response of the recently identified polyamine site by acting at a separate Con-G (SEQ ID NO: 1) site, and that derivatives of Con-G (SEQ ID NO: 1) modulate a polyamine associated site now allows an additional level of control to be exerted over the actions of the NMDA receptor. Clearly, Con-G (SEQ ID NO: 1) and its derivatives represent new classes of antagonists and partial agonists (functional antagonists) which modulate NMDA receptor function. Since Con-G (SEQ ID NO: 1) and its derivatives modulate the same function ($Ca^{++}$ influx) of the same receptor as compounds of the prior art, Con-G (SEQ ID NO: 1) and its derivatives should be equally useful in treating the broad spectrum of neuropsychopharmacological disorders and enhancement of CNS function as compounds of the prior art. In fact, the range of modulation presented from total antagonism through partial agonism (functional antagonism) to full agonism of the NMDA receptor by Con-G (SEQ ID NO: 1) and its derivatives represents far greater control of NMDA receptor function than is possible with the known modulators of the glutamate and glycine sites of the prior art. Thus, Con-G (SEQ ID NO: 1) and its derivatives should possess a broader range of efficacies for the treatment of CNS disorders and CNS function enhancement than is possible with compounds of the prior art. Recent evidence indicates that certain types of drug induced convulsions are also associated with chemical toxicity affecting the NMDA receptor. Greater control over the modulation of the NMDA receptor as is possible with the compounds of the present invention may present one of the best hopes for dealing with one of the less tractable current societal problems.

Although the invention has been described with reference to Con-G (SEQ ID NO: 1) and specific Con-G derivatives, the details should not be construed as limitations on the invention. Rather, various equivalents, modifications, and other derivatives as would be obvious to one skilled in the art upon learning of the invention and without departing from the spirit and scope of the invention are intended to be included within the scope of the disclosure and of the following claims.

References Cited

1. Foster, A. and Fagg, G. (1987) Taking apart NMDA receptors. Nature 329:395-396

2. Haack, J., Rivier, J., Parks, T., Mena, E., Cruz, L., and Olivera, B. (1990) Conantokin-T (A $\gamma$-carboxyglutamate containing peptide with N-methyl-D-aspartate antagonist activity). J. Biol. Chem. 265:6025-6029.

3. Johnson, K. and Jones, S. (1990) Neuropharmacology of Phencyclidine: Basic Mechanisms and Therapeutic Potential. Annu. Rev. Pharmacol. Toxicot. 30:707-750

4. Mayer, M. and Miller, R. (1990) Excitatory amino acid receptors, second messengers and regulation of intracellular $Ca^{2+}$ in mammalian neurons. Trends in Pharmacol. Sci. 11:254-260.

5. Mena, E., Gullak, M., Pagnozzi, M., Richter, K., Rivier, J., Cruz, L. and Olivera, B. (1990) Conantokin-G: a novel peptide antagonist to the N-methyl-D-aspartic acid (NMDA) receptor. Neurosci. Let. 118:241-244.

6. Olivera, B., Rivier, J., Clark, C., Ramilo, C., Corpuz, G., Abogadie, F., Mena, E., Woodward, S., Hillyard, D. and Cruz, L. (1990) Diversity of *Conus* neuropeptides. Science 249:257-263.

7. Ransom, R. and Stec, N. (1988) Cooperative modulation of [$^3$H]MK-801 binding to the N-methyl-D-aspartate receptor-ion channel complex by L-glutamate, glycine, and polyamines. J. Neurochem. 51:830-836.

8. Reynolds, I. J. and Miller, R. J. (1989) Ifenprodil is a novel type of N-methyl-D-aspartate receptor antagonist: interaction with polyamines. Molec. Pharmacol. 36:758-765.

9. Williams, K., Romano, C., Dichter, M., and Molinoff, P. (1991) Modulation of the NMDA Receptor by Polyamines. Life Sci. 48:469-498

The content of the paper copy of the Sequence Listing which follows and the computer readable copy (CRF) provided on disk are the same.

## Claims

1. A peptide selected from the group consisting of:

    (i)    Tyr-Gly-Glu-Gla-Gla-Leu-Gln-Gla-Asn-Gln-Gla-Leu-Ile-Arg-Gla-Lys-Ser-Asn-NH$_2$ (SEQ ID NO:3);

    (ii)    Gly-Glu-Glu-Glu-Leu-Gln-Glu-Asn-Gln-Glu-Leu-Ile-Arg-Glu-Lys-Ser-Asn-NH$_2$ (SEQ ID NO:5);

    (iii)    Tyr-Gly-Glu-Glu-Glu-Leu-Gln-Glu-Asn-Gln-Glu-Leu-Ile-Arg-Glu-Lys-Ser-Asn-NH$_2$ (SEQ ID NO:6);

    (iv)    Ile-Arg-Glu-Lys-Ser-Asn-NH$_2$ (SEQ ID NO:7);

(v)     Gln-Glu-Leu-Ile-Arg-Glu-Lys-Ser-Asn-NH$_2$ (SEQ ID NO:8);

(vi)     Gln-Glu-Asn-Gln-Glu-Leu-Ile-Arg-Glu-Lys-Ser-Asn-NH$_2$    (SEQ    ID    NO:9);

(vi)     Gln-Glu-Asn-Gln-Glu-Leu-Ile-Arg-Glu-Lys-Ser-Asn-NH$_2$    (SEQ    ID    NO:9);

(vii)     Glu-Glu-Glu-Leu-Gln-Glu-Asn-Gln-Glu-Leu-Ile-Arg-Glu-Lys-Ser-Asn-NH$_2$ (SEQ ID NO:10);

(viii)     Gly-Glu-DGlu-DGlu-Leu-Gln-Glu-Asn-Gln-DGlu-Leu-Ile-Arg-DGlu-Lys-Ser-Asn-NH$_2$ (SEQ ID NO:11).

2.    A pharmaceutical composition comprising the peptide of claim 1 and a pharmaceutically acceptable carrier.

3.    Use of a peptide as claimed in claim 1 in the manufacture of a medicament for the treatment of a neuropsychopharmacological disorder, a neurodegenerative disorder, a chemical toxicity or a cognitive deficit.

4.    Use of a peptide as set forth in claim 1 for the manufacture of a medicament for the enhancement of memory and associated mental processes.

5.    Use of Con-G (SEQ ID NO:1) in the manufacture of a medicament for treating excitotoxicity resulting from overstimulation of the NM13A receptor.

6.    Use according to claim 5, wherein said excitotoxicity is associated with epilepsy or epileptic seizure.

**Patentansprüche**

1.    Peptid, welches ausgewählt ist aus der Gruppe bestehend aus

(i)     Tyr-Gly-Glu-Gla-Gla-Leu-Gln-Gla-Asn-Gln-Gla-Leu-Ile-Arg-Gla-Lys-Ser-Asn-NH$_2$ (SEQ ID NO:3);

(ii)     Gly-Glu-Glu-Glu-Leu-Gln-Glu-Asn-Gln-Glu-Leu-Ile-Arg-Glu-Lys-Ser-Asn-NH$_2$ (SEQ ID NO:5);

(iii)     Tyr-Gly-Glu-Glu-Glu-Leu-Gln-Glu-Asn-Gln-Glu-Leu-Ile-Arg-Glu-Lys-Ser-Asn-NH$_2$ (SEQ ID NO:6);

EP 0 625 988 B1

(iv)     Ile-Arg-Glu-Lys-Ser-Asn-NH₂ (SEQ ID NO:7);

(iv)     Ile-Arg-Glu-Lys-Ser-Asn-NH₂ (SEQ ID NO:7);

(v)      Gln-Glu-Leu-Ile-Arg-Glu-Lys-Ser-Asn-NH₂ (SEQ ID NO:8);

(vi)     Gln-Glu-Asn-Gln-Glu-Leu-Ile-Arg-Glu-Lys-Ser-Asn-NH₂  (SEQ  ID NO:9);

(vii)    Glu-Glu-Glu-Leu-Gln-Glu-Asn-Gln-Glu-Leu-Ile-Arg-Glu-Lys-Ser-Asn-NH₂ (SEQ ID NO:10);

(viii)   Gly-Glu-DGlu-DGlu-Leu-Gln-Glu-Asn-Gln-DGlu-Leu-Ile-Arg-DGlu-Lys-Ser-Asn-NH₂ (SEQ ID NO:11).

**2.** Pharmazeutische Zusammensetzung umfassend das Peptid gemäß Anspruch 1 und eine pharmazeutisch annehmbare Trägersubstanz.

**3.** Verwendung eines Peptids nach Anspruch 1 zur Herstellung eines Arzneimittels zur Behandlung eines neuropsychopharmakologischen Krankheitszustands, eines neurodegenerativen Krankheitszustands, einer chemischen Toxizität oder eines kognitiven Defizits.

**4.** Verwendung eines Peptids nach Anspruch 1 zur Herstellung eines Arzneimittels zur Förderung des Gedächtnisses und von damit verbundenen mentalen Prozessen.

**5.** Verwendung von Con-G (SEQ ID NO:1) zur Herstellung eines Arzneimittels zur Behandlung von Excitotoxizität, welche von einer Überstimulierung des NM13A Rezeptors verursacht ist

**6.** Verwendung nach Anspruch 5, worin die Excitotoxizität mit einer Epilepsie oder einem epileptischen Anfall einhergeht.

**Revendications**

**1.** Peptide sélectionné dans le groupe constitué de

(i)      Tyr-Gly-Glu-Gla-Gla-Leu-Gln-Gla-Asn-Gln-Gla-Leu-Ile-Arg-Gla-Lys-Ser-Asn-NH₂ (SEQ ID NO:3);

(ii)     Gly-Glu-Glu-Glu-Leu-Gln-Glu-Asn-Gln-Glu-Leu-Ile-Arg-Glu-Lys-Ser-Asn-NH₂ (SEQ ID NO:5);

(iii)    Tyr-Gly-Glu-Glu-Glu-Leu-Gln-Glu-Asn-Gln-Glu-Leu-Ile-Arg-Glu-Lys-Ser-Asn-NH₂ (SEQ ID NO:6);

(iv)  Ile-Arg-Glu-Lys-Ser-Asn-NH$_2$ (SEQ ID NO:7);

(v)  Gln-Glu-Leu-Ile-Arg-Glu-Lys-Ser-Asn-NH$_2$ (SEQ ID NO:8);

(vi)  Gln-Glu-Asn-Gln-Glu-Leu-Ile-Arg-Glu-Lys-Ser-Asn-NH$_2$ (SEQ ID NO:9);

(vii)  Glu-Glu-Glu-Leu-Gln-Glu-Asn-Gln-Glu-Leu-Ile-Arg-Glu-Lys-Ser-Asn-NH$_2$ (SEQ ID NO:10);

(viii)  Gly-Glu-DGlu-DGlu-Leu-Gln-Glu-Asn-Gln-DGlu-Leu-Ile-Arg-DGlu-Lys-Ser-Asn-NH$_2$ (SEQ ID NO:11).

2. Composition pharmaceutique comportant le peptide suivant la revendication 1 et un porteur acceptable pharmaceutiquement.

3. Utilisation d'un peptide suivant la revendication 1 pour la fabrication d'un médicament pour le traitement d'un trouble neuro psychopharmacologique, d'un trouble neuro dégénératif, d'une toxicité chimique ou d'un déficit cognitif.

4. Utilisation d'un peptide suivant la revendication 1, pour la fabrication d'un médicament pour l'amélioration de la mémoire et des processus mentaux associés.

5. Utilisation de Con-G (SEQ ID NO:1) pour la fabrication d'un médicament pour traiter l'excito-toxicité résultant d'une surstimulation du récepteur NM13A.

6. Utilisation suivant la revendication 5. dans laquelle l'excito-toxicité est associée à l'épilepsie ou attaque épileptique.

FIG.1

FIG.2B

FIG.2A

FIG.3A

FIG.3B

FIG.4

FIG.5

FIG.6

FIG.7

FIG.8

Glu Con-G (12-17)

Glu Con-G

N Ac Con-G

Tyr⁰ Con-G

Con-G

Con-G-OH

[³H] MK-801 BOUND % SPERMINE MAX

[ ] μM

FIG.9

FIG.10

EP 0 625 988 B1

FIG.11A

FIG.11B

35

FIG.12

FIG.13

FIG.14A

FIG.14B

EP 0 625 988 B1

FIG.15

FIG.17

| TABLE I | | | |
|---|---|---|---|
| ENHANCEMENT OF MK–801 BINDING | | | |
| COMPOUND | POTENCY 1/2 STIMULATION $EC_{50}, \mu M$ | EFFICACY MAXIMUM STIMULATION | EFFICACY AS % OF $SPERMINE_{max}$ |
| BASAL BINDING | NO ACTIVITY | 20.0 | 0 |
| SPERMINE | 8.6 | 159.8 | 100 |
| SPERMIDINE | 10.8 | 155.1 | 97 |
| $Mg^{2+}$ | 20.6 | 82.7 | 52 |
| $Tyr^0$–Con G | 1.3 | 114.0 | 72 |
| Glu–Con G | 6.7 | 126.8 | 79 |
| D Glu–Con G | 5.8 | 47.8 | 30 |
| Glu–ConG (12–17) | 52.0 | 113.1 | 74 |
| Glu–ConG (9 – 17) | MARGINAL ACTIVITY | 29.0 | 18 |
| Glu–ConG (6 – 17) | MARGINAL ACTIVITY | 25.0 | 16 |
| Glu–ConG (2 – 17) | 0.91 | 51.8 | 33 |
| $Tyr^0$, Glu–ConG | 2.7 | 48.3 | 31 |
| NAc–Con G | MARGINAL ACTIVITY | 26.0 | 16 |
| Con G–OH | NO ACTIVITY | NO ACTIVITY | 0 |

FIG.16